# EUROPEAN PATENT SPECIFICATION

(11) **EP 1 173 201 B1**
(45) Date of publication and mention of the grant of the patent: **22.06.2005**
(21) Application number: 00926238.7
(22) Date of filing: 21.04.2000
(51) Int. Cl.: A61K 39/395, A61P 11/00, A61P 37/00

(54) **METHOD FOR THE TREATMENT OF FIBROSIS USING AN ANTAGONIST OF THE INTEGRIN ALPHA-4 SUBUNIT**
VERFAHREN ZUR BEHANDLUNG EINER FIBROSE UNTER VERWENDUNG EINES ANTAGONISTEN DER INTEGRIN ALPHA-4 UNTEREINHEIT
METHODE DE TRAITEMENT DE LA FIBROSE UTILISANT UN ANTAGONISTE DE LA SOUS-UNITE ALPHA-4 DE L'INTEGRINE

(30) Priority: 22.04.1999 US 130847 P; 01.06.1999 US 137214 P
(43) Date of publication of application: 23.01.2002
(60) Divisional application: 05007878.1
(73) Proprietor: Biogen Idec MA Inc., Cambridge, Massachusetts 02142 (US)
(72) Inventor: GOTWALS, Philip, West Roxbury, MA 02132 (US); LOBB, Roy, R., Westwood, MA 02090 (US)
(74) Representative: VOSSIUS & PARTNER
(86) International application number: PCT/US2000/010781
(87) International publication number: WO 2000/064474

(56) References cited:
- WO-A-93/13798
- WO-A-94/17828
- WO-A-95/19790
- WO-A-97/11718
- WO-A-97/18838
- T. PAPADOPOULOS ET AL.: "Expressionsmuster von Integrinen auf Alveolarepithelien in der fetalen und adulten Lunge und bei interstitiellen Lungenerkrankungen (Pattern of expression of integrins in alveolar epithelia of fetal and adult lungs and interstitial lung diseases)." VERHANDLUNGEN DER DEUTSCHEN GESELLSCHAFT FÜR PATHOLOGIE, vol. 77, 1993, pages 292-295, XP000929345 Stuttgart, Germany
- S. HOKIBARA ET AL.: "Effects of monoclonal antibodies to adhesion molecules on eosinophilic myocarditis in Toxocara canis-infected CBA/J mice." CLINICAL AND EXPERIMENTAL IMMUNOLOGY, vol. 114, no. 2, November 1998 (1998-11), pages 236-244, XP000929346 Oxford, GB
- P. ROY-CHAUDHURY ET AL.: "Adhesion molecule interactions in human glomerulonephritis: importance of the tubulointerstitium." KIDNEY INTERNATIONAL, vol. 49, no. 1, January 1996 (1996-01), pages 127-134, XP000929347 New York, NY, USA
- K. LIN ET AL.: "Very late antigen 4 (VLA4) antagonists as anti-inflammatory agents." CURRENT OPINION IN CHEMICAL BIOLOGY, vol. 2, no. 4, August 1998 (1998-08), pages 453-457, XP000929360 London, GB
- R. LOBB ET AL.: "The pathophysiologic role of alpha-4 integrins in vivo." JOURNAL OF CLINICAL INVESTIGATION, vol. 94, no. 5, November 1994 (1994-11), pages 1722-1728, XP000929359 New York, NY, USA
- R. LOBB ET AL.: "The role of alpha-4 integrins in lung pathophysiology." EUROPEAN RESPIRATORY JOURNAL, SUPPLEMENT, vol. 9, no. suppl. 22, August 1996 (1996-08), pages 104S-108S, XP000929340 Copenhagen, Denmark

## Description

### BACKGROUND OF THE INVENTION

Fibronectin and collagen are proteins which are essential for maintaining the integrity of the extracellular matrix found in connective tissues. The production of these proteins is a highly regulated process, and its disturbance may lead to the development of tissue fibrosis. While the formation of fibrous tissue is part of the normal beneficial process of healing after injury, in some circumstances there is an abnormal accumulation of fibrous materials such that it may ultimately lead to organ failure (Border et al. (1994) *New Engl. J. Med*. 331:1286-1292). Injury to any organ leads to a stereotypical physiological response: platelet-induced hemostasis, followed by an influx of inflammatory cells and activated fibroblasts. Cytokines derived from these cell types drive the formation of new extracellular matrix and blood vessels (granulation tissue). The generation of granulation tissue is a carefully orchestrated program in which the expression of protease inhibitors and extracellular matrix proteins is upregulated, and the expression of proteases is reduced, leading to the accumulation of extracellular matrix.

Central to the development of fibrotic conditions, whether induced or spontaneous, is stimulation of fibroblast activity. The influx of inflammatory cells and activated fibroblasts into the injured organ depends on the ability of these cell types to interact with the interstitial matrix comprised primarily of fibronectin and collagen. These cell-cell or cell-extracellular matrix interactions are mediated through several families of cell adhesion molecules, one such family of which includes the integrins. Integrins are structurally and functionally related glycoproteins consisting of various alpha (alpha1, alpha 2, up to alpha 11 at present) and beta (beta 1 and beta 7) heterodimeric transmembrane receptor domains found in various combinations on virtually every mammalian cell type. (for reviews see: E. C. Butcher, Cell, 67, 1033 (1991); D. Cox et al., "The Pharmacology of the Integrins." Medicinal Research Rev. Vol. 195 (1994) and V. W. Engleman et al., "Cell Adhesion Integrins as Pharmaceutical Targets" in Ann, Revs.Medicinal Chemistry, Vol. 31, J. A. Bristol, Ed.; Acad. Press, NY, 1996, p. 191). Two alpha4 subunit containing integrins have been described and are designated alpha4betal (VLA-4) and alpha4beta7.

Interstitial pulmonary fibrosis (IPF) is the final pathway of many interstitial lung diseases that leads to a reduction of lung compliance and impairment of the vital gas exchange function. Regardless of the etiology, IPF is characterized by inflammatory and fibroproliferative changes of the lung and an excess accumulation of collagen in the interstitium. Patients with IPF generally demonstrate a presence of recruited immune and inflammatory cells during active Pulmonary fibrosis indicating that pulmonary fibrosis is the results of aberrant repair after an initial inflammatory insult. Recruited inflammatory cells are likely to be involved in the initial insult in many instances. In addition, these cells may play a complex role in regulating the repair process. In either case, the recruitment of immune and inflammatory cells into the lung may play an important role in the determination of the fibrotic response. The influx of inflammatory cells and activated fibroblasts into the injured lung depends on the ability of these cell types to interact with components of the ECM. The traffic and state of activation of leukocytes are modulated by various integrins. The prevention of the influx of inflammatory cells into lungs may be critical in containing the subsequent fibrotic response.

Many of the diseases associated with the proliferation of fibrous tissue are both chronic and often debilitating, including for example, skin diseases such as scleroderma. Some, including pulmonary fibrosis, can be fatal due in part to the fact that the currently available treatments for this disease have significant side effects and are generally not efficacious in slowing or halting the progression of fibrosis [Nagler et al. 1996, *Am. J. Respir. Crit. Care Med,* 154:1082-86]. There is, accordingly, a continuing need for new anti-fibrotic agents.
-- WO-A-97 18838 describes methods of treatment using humanized immunoglobulins that specifically bind to alpha-4 integrin and that can be used in the treatment of disorders such as acute leukocyte mediated lung injury (p. 25).--
-- WO 97/11718 discloses the use of a neutralizing antibody that is specific to the RGD recognition site of integrins and that may be a neutralizing antibody specific to the integrin receptor.--
-- Kidney International, 1996, 49:127-134 discloses that there is not a strong association between glomerular expression of LFA-1, VLA-4, ICAM-1, VCAM-1, and glomerular macrophage infiltration and chronic histological damage.--

### SUMMARY OF THE INVENTION

The present invention provides antagonists of an interaction between an alpha4 subunit-bearing integrin for the preparation of a pharmaceutical composition useful for treating fibrosis in a subject. We have investigated the possible role of antagonists of alpha1 and alpha4 subunit-containing integrins in the pathogenesis of fibrosis by administering an antagonist of an alpha1 or alpha4 subunit containing integrin to mice with lung fibrosis. The beneficial effect that these antagonists have on both total collagen accumulation and the extent of pulmonary fibrotic lesions as shown in the present communication suggest that the alphal and/or alpha4 subunit containing integrins may be a reasonable target for antifibrotic therapy. One aspect of the invention is the use of a composition comprising an antagonist of an interaction between an alpha4 subunit-bearing integrin and a ligand for an alpha4 subunit-bearing integrin for the preparation of a pharmaceutical composition for the treatment of a subject having a fibrotic condition. The antagonist is an alpha 4 integrin binding agent or an alpha4 integrin ligand binding agent. Preferred alpha 4 integrin binding agents are selected from the group consisting of; a) an antibody homolog that antagonizes the interaction of both VLA-4 and alpha4 beta 7 with their respective alpha4 ligands; b) an antibody homolog that antagonizes the interaction of VLA-4 with its alpha4 ligand; and c) an antibody homolog that antagonizes the interaction of alpha4beta7 with its alpha4 ligand. In other embodiments, the antibody homolog is selected from the group consisting of a human antibody, a chimeric antibody, a humanized antibody and fragments thereof.

Another aspect of the present invention is the use of an antagonist of an interaction between an alpha4 subunit-bearing integrin and a ligand for an alpha4 subunit-bearing integrin for the preparation of a pharmaceutical composition for decreasing fibrotic condition-induced increases in leukocytes in a sample of bronchoalveolar lavage fluid of a subject having a fibrotic condition.

In certain embodiments of the invention, the alpha 4 integrin binding agent is encoded by a nucleic acid sequence comprising a nucleic acid that hybridizes under several stringency conditions to defined nucleic acid sequences selected from the group of sequences in Table 6 of U.S. Patent 5,840,299 or the complement of said nucleic acid sequences. In other aspects of the method, the alpha 4 integrin binding agent is encoded by a nucleic acid sequence comprising a nucleic acid that hybridizes under defined stringency conditions to a nucleic acid sequence encoding a polypeptide sequence selected from the group consisting of certain defined polypeptides found in U.S. Patent 5,932,214 or produced by cell line ATCC CRL 11175.

### DETAILED DESCRIPTION OF THE INVENTION

### I. Definitions

In order to more clearly and concisely point out the subject matter of the claimed invention, the following definitions are provided for specific terms used in the following written description and appended claims.

The invention will now be described with reference to the following detailed description of which the following definitions are included:

The integrin very late antigen (VLA) superfamily is made up of structurally and functionally related glycoproteins consisting of (alpha and beta) heterodimeric, transmembrane receptor molecules found in various combinations on nearly every mammalian cell type. (for reviews see: E. C. Butcher, Cell, 67, 1033 (1991); D. Cox et al., "The Pharmacology of the Integrins." Medicinal Research Rev. (1994) and V. W. Engleman et al., Cell Adhesion Integrins as Pharmaceutical Targets.' in Ann. Report in Medicinal Chemistry, Vol. 31, J. A. Bristol, Ed.; Acad. Press, NY, 1996, p. 191). Integrins of the VLA family include (at present) VLA-1, -2, -3, -4, -5, -6, -9, and -11 in which each of the molecules comprise a β1 chain non- covalently bound to an alpha chain, (α1, α2, α3, α4, α5, α6 and the like), respectively.

Alpha 4 beta 1 (α4β1) integrin is a cell-surface receptor for VCAM-1, fibronectin and possibly other ligands (the latter ligands individually and collectively referred to as "alpha4 ligand(s)"). The term α4β1 integrin ("VLA-4" or "a4b1" or "alpha4beta1 integrin", used interchangeably) herein thus refers to polypeptides which are capable of binding to VCAM-1 and members of the extracellular matrix proteins, most particularly fibronectin, or homologs or fragments thereof, although it will be appreciated by workers of ordinary skill in the art that other ligands for VLA-4 may exist and can be analyzed using conventional methods. Nevertheless, it is known that the alpha4 subunit will associate with other beta subunits besides beta1 so that we may define the term "alpha (α) 4 integrin" or "alpha (α) 4 subunit-containing integrin" as being those integrins whose alpha4 subunit associates with one or another of the beta subunits. Another example of an "alpha4" integrin besides VLA4 is alpha4beta7 (See Lobb and Adams, supra). Similarly, an 'alpha1 integrin" or 'alpha1 subunit-containing integrin' are those integrins whose alpha1 subunit associates with one or another of the beta subunits.

An integrin "antagonist" includes any compound that inhibits alphal and/or alpha4 subunit-containing integrins from binding with an integrin ligand and/or receptor. Anti-integrin antibody or antibody homolog-containing proteins (discussed below) as well as other molecules such as soluble forms of the ligand proteins for integrins are useful. Soluble forms of the ligand proteins for alpha4 subunit-containing integrins include soluble VCAM-1, VCAM-1 fusion proteins, or bifunctional VCAM-1/Ig fusion proteins. For example, a soluble form of an integrin ligand or a fragment thereof may be administered to bind to integrin, and preferably compete for an integrin binding site on cells, thereby leading to effects similar to the administration of antagonists such as anti-integrin (e.g., VLA-1, VLA-4) antibodies. In particular, soluble integrin mutants that bind ligand but do not elicit integrin-dependent signaling are included within the scope of the invention. Such integrin mutants can act as competitive inhibitors of wild type integrin protein and are considered "antagonists". Other antagonists used in the methods of the invention are "small molecules", as defined below.

Also included within the invention is the use of molecules that antagonize the action of more than one alpha 4 subunit-containing integrin, such as small molecules or antibody homologs that antagonize both VLA-4 and alpha4 beta7 or other combinations of alpha4 subunit-containing integrins. Also included is the use of molecules that antagonize the action of more than one alpha 1 subunit-containing integrin. Also included within the scope of the invention is the use of a combination of molecules such that the combination antagonizes the action of more than one integrin, such as the use of several small molecules or antibody homologs that in combination antagonize both VLA-4 and alpha4 beta7 or other combinations of alpha4 subunit-containing integrins.

As discussed herein, certain integrin antagonists can be fused or otherwise conjugated to, for instance, an antibody homolog such as an immunoglobulin or fragment thereof and are not limited to a particular type or structure of an integrin or ligand or other molecule. Thus, for purposes of the invention, any agent capable of forming a chimeric protein (as defined below) and capable of binding to integrin ligands and which effectively blocks or coats alpha4 and/or alpha1 subunit containing integrin is considered to be an equivalent of the antagonists used in the examples herein.

"antibody homolog" includes intact antibodies consisting of immunoglobulin light and heavy chains linked via disulfide bonds. The term "antibody homolog" is also intended to encompass a protein comprising one or more polypeptides selected from immunoglobulin light chains, immunoglobulin heavy chains and antigen-binding fragments thereof which are capable of binding to one or more antigens (i.e., integrin or integrin ligand). The component polypeptides of an antibody homolog composed of more than one polypeptide may optionally be disulfide-bound or otherwise covalently crosslinked. Accordingly, therefore, "antibody homologs" include intact immunoglobulins of types IgA, IgG, IgE, IgD, IgM (as well as subtypes thereof), wherein the light chains of the immunoglobulin may be of types kappa or lambda. "Antibody homologs" also includes portions of intact antibodies that retain antigen-binding specificity, for example Fab fragments, Fab' fragments, F(ab')2 fragments, F(v) fragments, heavy and light chain monomers or dimers or mixtures thereof.

"humanized antibody homolog" is an antibody homolog, produced by recombinant DNA technology, in which some or all of the amino acids of a human immunoglobulin light or heavy chain that are not required for antigen binding have been substituted for the corresponding amino acids from a nonhuman mammalian immunoglobulin light or heavy chain. A "human antibody homolog" is an antibody homolog in which all the amino acids of an immunoglobulin light or heavy chain (regardless of whether or not they are required for antigen binding) are derived from a human source.

As used herein, a "human antibody homolog" is an antibody homolog produced by recombinant DNA technology, in which all of the amino acids of an immunoglobulin light or heavy chain that are derived from a human source.

An integrin "agonist" includes any compound that activates the integrin ligand.
"amino acid" is a monomeric unit of a peptide, polypeptide, or protein. There are twenty amino acids found in naturally occurring peptides, polypeptides and proteins, all of which are L-isomers. The term also includes analogs of the amino acids and D-isomers of the protein amino acids and their analogs.
"covalently coupled" - means that the specified moieties of the invention (e.g., PEGylated alpha 4 and/or alpha1 integrin antagonist, immunoglobulin fragment/ alpha 4 or alpha 1 integrin antagonist) are either directly covalently bonded to one another, or else are indirectly covalently joined to one another through an intervening moiety or moieties, such as a spacer moiety or moieties. The intervening moiety or moieties are called a "coupling group". The term "conjugated" is used interchangeably with "covalently coupled". In this regard a "spacer" refers to a moiety that may be inserted between an amino acid or other component of an integrin antagonist or fragment and the remainder of the molecule. A spacer may provide separation between the amino acid or other component and the rest of the molecule so as to prevent the modification from interfering with protein function and/or make it easier for the amino acid or other component to link with another moiety.
"expression control sequence"- a sequence of polynucleotides that controls and regulates expression of genes when operatively linked to those genes.
"expression vector"- a polynucleotide, such as a DNA plasmid or phage (among other common examples) which allows expression of at least one gene when the expression vector is introduced into a host cell. The vector may, or may not, be able to replicate in a cell.

An "effective amount" of an agent of the invention is that amount which produces a result or exerts an influence on the particular condition being treated.
"functional equivalent" of an amino acid residue is (i) an amino acid having similar reactive properties as the amino acid residue that was replaced by the functional equivalent; (ii) an amino acid of an antagonist of the invention, the amino acid having similar properties as the amino acid residue that was replaced by the functional equivalent; (iii) a non-amino acid molecule having similar properties as the amino acid residue that was replaced by the functional equivalent.

A first polynucleotide encoding a proteinaceous antagonist of the invention is "functionally equivalent" compared with a second polynucleotide encoding the antagonist protein if it satisfies at least one of the following conditions:
(a): the "functional equivalent" is a first polynucleotide that hybridizes to the second polynucleotide under standard hybridization conditions and/or is degenerate to the first polynucleotide sequence. Most preferably, it encodes a mutant protein having the activity of an integrin antagonist protein;
(b) the "functional equivalent" is a first polynucleotide that codes on expression for an amino acid sequence encoded by the second polynucleotide.

The integrin antagonists used in the invention include, but are not limited to, the agents listed herein as well as their functional equivalents. As used herein, the term "functional equivalent" therefore refers to an integrin antagonist or a polynucleotide encoding the integrin antagonist that has the same or an improved beneficial effect on the recipient as the integrin antagonist of which it is deemed a functional equivalent. As will be appreciated by one of ordinary skill in the art, a functionally equivalent protein can be produced by recombinant techniques, e.g., by expressing a "functionally equivalent DNA". Accordingly, the instant invention embraces integrin proteins encoded by naturally-occurring DNAs, as well as by non-naturally-occurring DNAs which encode the same protein as encoded by the naturally-occurring DNA. Due to the degeneracy of the nucleotide coding sequences, other polynucleotides may be used to encode integrin protein. These include all, or portions of the above sequences which are altered by the substitution of different codons that encode the same amino acid residue within the sequence, thus producing a silent change. Such altered sequences are regarded as equivalents of these sequences. For example, Phe (F) is coded for by two codons, TTC or TTT, Tyr (Y) is coded for by TAC or TAT and His (H) is coded for by CAC or CAT. On the other hand, Trp (W) is coded for by a single codon, TGG. Accordingly, it will be appreciated that for a given DNA sequence encoding a particular integrin there will be many DNA degenerate sequences that will code for it. These degenerate DNA sequences are considered within the scope of this invention.

The term "chimeric" when referring to an antagonist of the invention, means that the antagonist is comprised of a linkage (chemical cross-linkage or covalent or other type) of two or more proteins having disparate structures and/or having disparate sources of origin. Thus, a chimeric alpha 4 integrin antagonist may include one moiety that is an alpha 4 integrin antagonist or fragment and another moiety that is not an alpha 4 integrin antagonist. A chimeric alpha 1 integrin antagonist may include one moiety that is an alpha 1 integrin antagonist or fragment and another moiety that is not an alpha 1 integrin antagonist.

A species of 'chimeric' protein is a "fusion" or "fusion protein" which refers to a co-linear, covalent linkage of two or more proteins or fragments thereof via their individual peptide backbones, most preferably through genetic expression of a polynucleotide molecule encoding those proteins. Thus, preferred fusion proteins are chimeric proteins that include an alpha4 (or alpha1) integrin antagonist or fragment covalently linked to a second moiety that is not an alpha 4 (or alpha 1) integrin antagonist. Preferred fusion proteins of the invention may include portions of intact antibodies that retain antigen-binding specificity, for example, Fab fragments, Fab' fragments, F(ab')2 fragments, F(v) fragments, heavy chain monomers or dimers, light chain monomers or dimers, dimers consisting of one heavy and one light chain, and the like.

The most preferred fusion proteins are chimeric and comprise an integrin antagonist moiety fused or otherwise linked to all or part of the hinge and constant regions of an immunoglobulin light chain, heavy chain, or both. Thus, this invention features a molecule which includes: (1) an integrin antagonist moiety, (2) a second peptide, e.g., one which increases solubility or in vivo life time of the integrin antagonist moiety, e.g., a member of the immunoglobulin super family or fragment or portion thereof, e.g., a portion or a fragment of IgG, e.g., the human IgGl heavy chain constant region, e.g., CH2, CH3, and hinge regions. Specifically, a "integrin antagonist/Ig fusion" is a protein comprising a biologically active integrin antagonist molecule of the invention (e.g. a soluble VLA-4 or VLA-1 ligand, or a biologically active fragment thereof linked to an N-terminus of an immunoglobulin chain wherein a portion of the N-terminus of the immunoglobulin is replaced with the integrin antagonist. A species of integrin antagonist/Ig fusion is an "integrin /Fc fusion" which is a protein comprising an integrin antagonist of the invention linked to at least a part of the constant domain of an immunoglobulin. A preferred Fc fusion comprises a integrin antagonist of the invention linked to a fragment of an antibody containing the C terminal domain of the heavy immunoglobulin chains.

The term "fusion protein" also means an integrin antagonist chemically linked via a mono- or hetero- functional molecule to a second moiety that is not an integrin antagonist (resulting in a "chimeric" molecule) and is made de novo from purified protein as described below. Thus, one example of a chemically linked, as opposed to recombinantly linked, chimeric molecule that is a fusion protein may comprise: (1) an alpha 4 integrin subunit targeting moiety, e.g., a VCAM-1 moiety capable of binding to VLA-4) on the surface of VLA-4 bearing cells; (2) a second molecule which increases solubility or in vivo life time of the targeting moiety, e.g., a polyalkylene glycol polymer such as polyethylene glycol (PEG). The alpha4 targeting moiety can be any naturally occurring alpha4 ligand or fragment thereof, e.g., a VCAM-1 peptide or a similar conservatively substituted amino acid sequence.

"Heterologous promoter"- as used herein is a promoter which is not naturally associated with a gene or a purified nucleic acid.

"Homology"- as used herein is synonymous with the term "identity" and refers to the sequence similarity between two polypeptides, molecules, or between two nucleic acids. When a position in both of the two compared sequences is occupied by the same base or amino acid monomer subunit (for instance, if a position in each of the two DNA molecules is occupied by adenine, or a position in each of two polypeptides is occupied by a lysine), then the respective molecules are homologous at that position. The percentage homology between two sequences is a function of the number of matching or homologous positions shared by the two sequences divided by the number of positions compared × 100. For instance, if 6 of 10 of the positions in two sequences are matched or are homologous, then the two sequences are 60% homologous. By way of example, the DNA sequences CTGACT and CAGGTT share 50% homology (3 of the 6 total positions are matched). Generally, a comparison is made when two sequences are aligned to give maximum homology. Such alignment can be provided using, for instance, the method of Karlin and Altschul described in more detail below.

Homologous sequences share identical or similar amino acid residues, where similar residues are conservative substitutions for, or "allowed point mutations" of, corresponding amino acid residues in an aligned reference sequence. In this regard, a "conservative substitution" of a residue in a reference sequence are those substitutions that are physically or functionally similar to the corresponding reference residues, e.g., that have a similar size, shape, electric charge, chemical properties, including the ability to form covalent or hydrogen bonds, or the like. Particularly preferred conservative substitutions are those fulfilling the criteria defined for an "accepted point mutation" in Dayhoff et al., 5: **Atlas of Protein Sequence and Structure,** 5: Suppl. 3, chapter 22: 354-352, Nat. Biomed. Res. Foundation, Washington, D.C. (1978).

"Homology" and "identity" are interchangeable herein and each refer to sequence similarity between two polypeptide sequences. Homology and identity can be determined by comparing a position in each sequence which may be aligned for purposes of comparison. When a position in the compared sequence is occupied by the same amino acid residue, then the polypeptides can be referred to as identical at that position; when the equivalent site is occupied by the same amino acid (e.g., identical) or a similar amino acid (e.g., similar in steric and/or electronic nature), then the molecules can be refered to as homologous at that position. A percentage of homology or identity between sequences is a function of the number of matching or homologous positions shared by the sequences. An "unrelated" or "non-homologous" sequence shares less than 40 percent identity, though preferably less than 25 percent identity, with a sequence of the present invention.

"Percent homology" of two amino acids sequences or two nucleic acid sequences is determined using the alignment algorithm of Karlin and Altschul (Proc. Nat. Acad. Sci., USA 87: 2264 (1990) as modified in Karlin and Altschul (Proc. Nat. Acad. Sci., USA 90: 5873 (1993). Such an algorithm is incorporated into the NBLAST or XBLAST programs of Altschul et al., J. Mol. Biol. 215: 403 (1990). BLAST searches are performed with the NBLAST program, score = 100, wordlength = 12, to obtain nucoetide sequences homologous to a nucleic acid of the invention. BLAST protein searches are performed with the XBLAST program, score = 50, wordlength = 3, to obtain amino acid sequences homologous to a reference polypeptide. To obtain gapped alignments for comparisons, gapped BLAST is used as described in Altschul et al., Nucleic Acids Res., 25: 3389 (1997). When using BLAST and Gapped BLAST, the default parameters of the respective programs (XBLAST and NBLAST) are used. See http://www/ncbi.nlm.nih.gov.

"Isolated" (used interchangeably with "substantially pure") when applied to nucleic acid i.e., polynucleotide sequences that encode integrin antagonists, means an RNA or DNA polynucleotide, portion of genomic polynucleotide, cDNA or synthetic polynucleotide which, by virtue of its origin or manipulation: (i) is not associated with all of a polynucleotide with which it is associated in nature (e.g., is present in a host cell as an expression vector, or a portion thereof); or (ii) is linked to a nucleic acid or other chemical moiety other than that to which it is linked in nature; or (iii) does not occur in nature. By "isolated" it is further meant a polynucleotide sequence that is: (i) amplified *in vitro* by, for example, polymerase chain reaction (PCR); (ii) synthesized chemically; (iii) produced recombinantly by cloning; or (iv) purified, as by cleavage and gel separation. Thus, "substantially pure nucleic acid" is a nucleic acid which is not immediately contiguous with one or both of the coding sequences with which it is normally contiguous in the naturally occurring genome of the organism from which the nucleic acid is derived. Substantially pure DNA also includes a recombinant DNA which is part of a hybrid gene encoding additional integrin sequences.

Isolated" (used interchangeably with "substantially pure")- when applied to polypeptides means a polypeptide or a portion thereof which, by virtue of its origin or manipulation: (i) is present in a host cell as the expression product of a portion of an expression vector; or (ii) is linked to a protein or other chemical moiety other than that to which it is linked in nature; or (iii) does not occur in nature, for example, a protein that is chemically manipulated by appending, or adding at least one hydrophobic moiety to the protein so that the protein is in a form not found in nature.. By "isolated" it is further meant a protein that is: (i) synthesized chemically; or (ii) expressed in a host cell and purified away from associated and contaminating proteins. The term generally means a polypeptide that has been separated from other proteins and nucleic acids with which it naturally occurs. Preferably, the polypeptide is also separated from substances such as antibodies or gel matrices (polyacrylamide) which are used to purify it.
"multivalent protein complex"- refers to a plurality of integrin antagonists (i.e., one or more). An anti-integrin antibody homolog or fragment may be cross-linked or bound to another antibody homolog or fragment. Each protein may be the same or different and each antibody homolog or fragment may be the same or different.
"mutant" - any change in the genetic material of an organism, in particular any change (i.e., deletion, substitution, addition, or alteration) in a wild type polynucleotide sequence or any change in a wild type protein. The term "mutein" is used interchangeably with "mutant".
"operatively linked"- a polynucleotide sequence (DNA, RNA) is operatively linked to an expression control sequence when the expression control sequence controls and regulates the transcription and translation of that polynucleotide sequence. The term "operatively linked" includes having an appropriate start signal (e.g., ATG) in front of the polynucleotide sequence to be expressed, and maintaining the correct reading frame to permit expression of the polynucleotide sequence under the control of the expression control sequence, and production of the desired polypeptide encoded by the polynucleotide sequence.

A "pharmacological agent", is defined as one or more compounds or molecules or other chemical entities administered to a subject (in addition to the antagonists of the invention) that affect the action of the antagonist. The term "pharmacological agent' as used herein refers to such an agent(s) that are administered during "combination therapy" where the antagonist of the invention is administered either prior to, after, or simultaneously with, administration of one or more pharmacological agents.
"protein"- any polymer consisting essentially of any of the 20 amino acids. Although "polypeptide" is often used in reference to relatively large polypeptides, and "peptide" is often used in reference to small polypeptides, usage of these terms in the art overlaps and is varied. The term "protein" as used herein refers to peptides, proteins and polypeptides, unless otherwise noted.

The terms "peptide(s)", "protein(s)" and "polypeptide(s)" are used interchangeably herein. The terms "polynucleotide sequence" and "nucleotide sequence" are also used interchangeably herein
"Recombinant," as used herein, means that a protein is derived from recombinant, mammalian expression systems. Since integrin is not glycosylated nor contains disulfide bonds, it can be expressed in most prokaryotic and eukaryotic expression systems.
"small molecule"- has the definition as in Section A2.

The phrase "surface amino acid" means any amino acid that is exposed to solvent when a protein is folded in its native form.
"hybridization conditions" generally mean salt and temperature conditions substantially equivalent to 0.5 X SSC to about 5 X SSC and 65 ° C for both hybridization and wash. The term "standard hybridization conditions" as used herein is therefore an operational definition and encompasses a range of hybridization conditions. Nevertheless, "high stringency" conditions include hybridizing with plaque screen buffer (0.2% polyvinylpyrrolidone, 0.2% Ficoll 400; 0.2% bovine serum albumin, 50 mM Tris-HCl (pH 7.5); 1 M NaCl; 0.1% sodium pyrophosphate; 1 % SDS); 10% dextran sulfate, and 100 µ g/ml denatured, sonicated salmon sperm DNA at 65 ° C for 12-20 hours, and washing with 75 mM NaCl/7.5 mM sodium citrate (0.5 x SSC)/1 % SDS at 65° C. "Low stringency" conditions include hybridizing with plaque screen buffer, 10% dextran sulfate and 110 µ g/ml denatured, sonicated salmon sperm DNA at 55 ° C for 12-20 hours, and washing with 300 mM NaCl/30mM sodium citrate (2.0 X SSC)/1% SDS at 55 ° C. See also **Current Protocols in Molecular Biology,** John Wiley & Sons, Inc. New York, Sections 6.3.1-6.3.6, (1989).

A "therapeutic composition" as used herein is defined as comprising the antagonists of the invention and other biologically compatible ingredients. The therapeutic composition may contain excipients such as water, minerals and carriers such as protein.

A "a subject with a fibrotic condition" refers to, but is not limited to, subjects afflicted with fibrosis of an internal organ, subjects afflicted with a dermal fibrosing disorder, and subjects afflicted with fibrotic conditions of the eye. Fibrosis of internal organs (e.g., liver, lung, kidney, heart blood vessels, gastrointestinal tract), occurs in disorders such as pulmonary fibrosis, myelofibrosis, liver cirrhosis, mesangial proliferative glomerulonephritis, crescentic glomerulonephritis, diabetic nephropathy, renal interstitial fibrosis, renal fibrosis in patients receiving cyclosporin, and HIV associated nephropathy. Dermal fibrosing disorders include, but are not limited to, scleroderma, morphea, keloids, hypertrophic scars, familial cutaneous collagenoma, and connective tissue nevi of the collagen type. Fibrotic conditions of the eye include conditions such as diabetic retinopathy, postsurgical scarring (for example, after glaucoma filtering surgery and after cross-eye surgery), and proliferative vitreoretinopathy. Additional fibrotic conditions which may be treated by the methods of the present invention include: rheumatoid arthritis, diseases associated with prolonged joint pain and deteriorated joints; progressive systemic sclerosis, polymyositis, dermatomyositis, eosinophilic fascitis, morphea, Raynaud's syndrome, and nasal polyposis. In addition, fibrotic conditions which may be treated the methods of present invention also include inhibiting overproduction of scarring in patients who are known to form keloids or hypertrophic scars, inhibiting or preventing scarring or overproduction of scarring during healing of various types of wounds including surgical incisions, surgical abdominal wounds and traumatic lacerations, preventing or inhibiting scarring and reclosing of arteries following coronary angioplasty, preventing or inhibiting excess scar or fibrous tissue formation associated with cardiac fibrosis after infarction and in hypersensitive vasculopathy.

An "effective amount" is an amount sufficient to effect beneficial or desired results. An effective amount can be administered in one or more administrations. In terms of treatment, an "effective amount" of an antagonist for use in the present invention is an amount sufficient to palliate, ameliorate, stabilize, reverse, slow or delay progression of a fibrotic condition in accordance with acceptable standards for disorders to be treated. Detection and measurement of indicators of efficacy may be measured by a number of available diagnostic tools, including, for example, by physical examination including blood tests, pulmonary function tests, and chest X-rays; CT scan; bronchoscopy; bronchoalveolar lavage; lung biopsy and CT scan.

Practice of the present invention will employ, unless indicated otherwise, conventional techniques of cell biology, cell culture, molecular biology, microbiology, recombinant DNA, protein chemistry, pharmacology and immunology, which are within the skill of the art. Such techniques are described in the literature.

### II. Description of the preferred Embodiments

The present application is directed to the discovery that-antagonists to alpha1 and/or alpha 4 subunit containing integrins and fragments thereof can be used for the treatment of pulmonary fibrosis.

### A. Integrin Antagonists

For the purposes of the invention an integrin antagonist can be an antagonist of any interaction between an integrin and its cognate ligand or receptor such that the normal function induced by ligand-receptor interactions is altered (i.e., prevented or slowed or otherwise modified). One preferred embodiment of an integrin antagonist is an antagonist of interactions of alpha4 integrins with their ligands, such as the VCAM-1/VLA-4 interaction. This is an agent, e.g., a polypeptide or other molecule, which can inhibit or block VCAM-1 and/or VLA-4-mediated binding or which can otherwise modulate VCAM-1 and/or VLA-4 function, e.g., by inhibiting or blocking VLA-4-ligand mediated VLA-4 signal transduction or VCAM-1-ligand mediated VCAM-1 signal transduction and which is effective in the treatment of acute brain injury, preferably in the same manner as are anti-VLA-4 antibodies.

An antagonist of the VCAM-1/ VLA-4 interaction is an agent which has one or more of the following properties: (1) it coats, or binds to, VLA-4 on the surface of a VLA-4 bearing cell (e.g., an endothelial cell) with sufficient specificity to inhibit a VLA-4-ligand/VLA-4 interaction, e.g., the VCAM-1/VLA-4 interaction; (2) it coats, or binds to, VLA-4 on the surface of a VLA-4 bearing cell (i.e., a lymphocyte) with sufficient specificity to modify, and preferably to inhibit, transduction of a VLA-4-mediated signal e.g., VLA-4/VCAM-1-mediated signaling; (3) it coats, or binds to, a VLA-4-ligand, (e.g., VCAM- 1) on endothelial cells with sufficient specificity to inhibit the VLA-4/VCAM-1 interaction; (4) it coats, or binds to, a VLA-4-ligand (e.g., VCAM- 1) with sufficient specificity to modify, and preferably to inhibit, transduction of VLA-4-ligand mediated VLA-4 signaling, e.g., VCAM-1-mediated VLA-4 signaling. In preferred embodiments the antagonist has one or both of properties 1 and 2. In other preferred embodiments the antagonist has one or both of properties 3 and 4. Moreover, more than one antagonist can be used e.g., an agent which binds to VLA-4 can be combined with an agent which binds to VCAM-1.

Another embodiment of an integrin antagonist is an antagonist of interactions of alpha1 integrins with their ligands, such as the collagen/VLA-1 interaction. This is an agent, e.g., a polypeptide or other molecule, which can inhibit or block collagen and/or VLA-1-mediated binding or which can otherwise modulate collagen and/or VLA-1 function, e.g., by inhibiting or blocking VLA-1-ligand mediated VLA-1 signal transduction or collagen- mediated collagen signal transduction. An antagonist of the collagen/ VLA-1 interaction is an agent which has one or more of the following properties: (1) it coats, or binds to, VLA-1 on the surface of a VLA-1 bearing cell (e.g., collagen) with sufficient specificity to inhibit a VLA-1-ligand/VLA-1 interaction, e.g., the collagen/VLA-1 interaction; (2) it coats, or binds to, VLA-1 on the surface of a VLA-1 bearing cell with sufficient specificity to modify, and preferably to inhibit, transduction of a VLA-1-mediated signal e.g., VLA-1/collagen-mediated signaling; (3) it coats, or binds to, a VLA-1-ligand, (e.g., collagen) with sufficient specificity to inhibit the VLA- 1/collagen interaction; (4) it coats, or binds to, a VLA-1-ligand with sufficient specificity to modify, and preferably to inhibit, transduction of VLA-1-ligand mediated VLA-1 signaling, e.g., collagen-mediated VLA-1 signaling. In preferred embodiments the alphal antagonist has one or both of properties 1 and 2. In other preferred embodiments the antagonist has one or both of properties 3 and 4. Moreover, more than one antagonist can be administered to a patient, e.g., an agent which binds to VLA-1 can be combined with an agent which binds to collagen.

As discussed herein, the antagonists used in the invention are not limited to a particular type or structure of molecule so that, for purposes of the invention and by way of example only, any agent capable of binding to alpha4 integrins (e.g., VLA-4) on the surface of cells or to an alpha4 ligand such as VCAM-1 on the surface of alpha4 ligand-bearing cells) and which effectively blocks or coats alpha 4 integrin (e.g., VLA-4) or alpha 4 ligand (e.g., VCAM-1), called an "alpha4 integrin binding agent" and "alpha4 integrin ligand binding agent" respectively), is considered to be an equivalent of the antagonists used in the examples herein.

For example, antibodies or antibody homologs (discussed below) as well as soluble forms of the natural binding proteins for VLA-4 and VCAM-1 are useful. Soluble forms of the natural binding proteins for VLA-4 include soluble VCAM-1 peptides, VCAM-1 fusion proteins, bifunctional VCAM-1/Ig fusion proteins (e.g. "chimeric" molecules, discussed above), fibronectin, fibronectin having an alternatively spliced non-type III connecting segment, and fibronectin peptides containing the amino acid sequence EILDV or a similar conservatively substituted amino acid sequence. Soluble forms of the natural binding proteins for VCAM-1 include soluble VLA-4 peptides, VLA-4 fusion proteins, bifunctional VLA-4/Ig fusion proteins and the like. As used herein, a "soluble VLA-4 peptide" or a "soluble VCAM-1 peptide" is an VLA-4 or VCAM-1 polypeptide incapable of anchoring itself in a membrane. Such soluble polypeptides include, for example, VLA-4 and VCAM polypeptides that lack a sufficient portion of their membrane spanning domain to anchor the polypeptide or are modified such that the membrane spanning domain is non-functional. These binding agents can act by competing with the cell-surface binding protein for VLA-4 or by otherwise altering VLA-4 function. For example, a soluble form of VCAM-1 (see, e.g., Osborn et al. 1989, Cell, 59: 1203-1211) or a fragment thereof may be administered to bind to VLA-4, and preferably compete for a VLA-4 binding site on VCAM-1-bearing cells, thereby leading to effects similar to the administration of antagonists such as small molecules or anti-VLA-4 antibodies.

### 1. Anti-Integrin Antibody Homologs

In other preferred embodiments, the antagonists used in the invention to bind to, including block or coat, cell-surface alpha1 and/or alpha4 integrin (such as VLA-1, VLA-4 or alpha4 beta7) and/or cell surface ligand for alpha1 and/or alpha 4 integrin (such as collagen or VCAM-1, respectively) is an anti-VLA-1 or anti-VLA-4 and/or anti-collagen and/or anti-VCAM-1 monoclonal antibody or antibody homolog, as defined previously. Preferred antibodies and homologs for treatment, in particular for human treatment, include human antibody homologs, humanized antibody homologs, chimeric antibody homologs, Fab, Fab', F(ab')2 and F(v) antibody fragments, and monomers or dimers of antibody heavy or light chains or mixtures thereof. Monoclonal antibodies against VLA4 are a preferred binding agent in the method of the invention.

### 2. Small Molecule Integrin Antagonists

The term " small molecule" integrin antagonist refers to chemical agents (i.e., organic molecules) capable of disrupting the integrin/integrin ligand interaction by, for instance, blocking VLA-4/VCAM interactions by binding VLA-4 on the surface of cells or binding VCAM-1 on the surface of cells. Such small molecules may also bind respective VLA-4 and VCAM-1 receptors. VLA-4 and VCAM-1 small molecule inhibitors may themselves be peptides, semi-peptidic compounds or non-peptidic compounds, such as small organic molecules that are antagonists of the VCAM-1/VLA-4 interaction. A "small molecule", as defined herein, is not intended to encompass an antibody or antibody homolog. The molecular weight of exemplary small molecules is generally less than 1000.

For instance, small molecules such as oligosaccharides that mimic the binding domain of a VLA-4 ligand and fit the receptor domain of VLA-4 may be employed. (See, J.J. Devlin et al., 1990, Science 249: 400-406 (1990), J.K. Scott and G.P. Smith, 1990, Science 249: 386-390, and U.S. Patent 4,833,092 (Geysen).

Conversely, small molecules that mimic the binding domain of a VCAM-1 ligand and fit the receptor domain of VCAM-1 may be employed.

Examples of other small molecules useful in the invention can be found in Komoriya et al. ("The Minimal Essential Sequence for a Major Cell Type-Specific Adhesion Site (CS1) Within the Alternatively Spliced Type III Connecting Segment Domain of Fibronectin Is Leucine-Aspartic Acid-Valine", J. Biol. Chem., 266 (23), pp. 15075-79 (1991)). They identified the minimum active amino acid sequence necessary to bind VLA4 and synthesized a variety of overlapping peptides based on the amino acid sequence of the CS-1 region (the VLA-4 binding domain) of a particular species of fibronectin. They identified an 8-amino acid peptide, Glu-Ile-Leu-Asp-Val-Pro-Ser-Thr, as well as two smaller overlapping pentapeptides, Glu-Ile-Leu-Asp-Val and Leu-Asp-Val-Pro-Ser, that possessed inhibitory activity against fibronectin-dependent cell adhesion. Certain larger peptides containing the LDV sequence were subsequently shown to be active in vivo (T. A. Ferguson et al., "Two Integrin Binding Peptides Abrogate T-cell-Mediated Immune Responses In Vivo", Proc. Natl. Acad. Sci. USA, 88, pp. 8072-76 (1991); and S. M. Wahl et al., "Synthetic Fibronectin Peptides Suppress Arthritis in Rats by Interrupting Leukocyte Adhesion and Recruitment", J. Clin. Invest., 94, pp. 655-62 (1994)). A cyclic pentapeptide, Arg-Cys-Asp-TPro-Cys (wherein TPro denotes 4-thioproline), which can inhibit both VLA-4 and VLA-5 adhesion to fibronectin has also been described. (See, e.g., D.M. Nowlin et al. "A Novel Cyclic Pentapeptide Inhibits Alpha4Beta1 Integrin-mediated Cell Adhesion", J. Biol. Chem., 268(27), pp. 20352-59 (1993); and PCT publication PCT/US91/04862). This pentapeptide was based on the tripeptide sequence Arg-Gly-Asp from fibronectin which had been known as a common motif in the recognition site for several extracellular-matrix proteins. Examples of other VLA-4 inhibitors have been reported, for example, in Adams et al. "Cell Adhesion Inhibitors", PCT US97/13013, describing linear peptidyl compounds containing beta-amino acids which have cell adhesion inhibitory activity. International patent applications WO 94/15958 and WO 92/00995 describe cyclic peptide and peptidomimetic compounds with cell adhesion inhibitory activity. International patent applications WO 93/08823 and WO 92/08464 describe guanidinyl-, urea- and thiourea-containing cell adhesion inhibitory compounds. United States Patent No. 5,260,277 describes guanidinyl cell adhesion modulation compounds. Other peptidyl antagonists of VLA-4 have been described in D. Y. Jackson et al., "Potent α4β1 peptide antagonists as potential anti-inflammatory agents', J. Med. Chem., 40,3359 (1997); H. Shroff et al., 'Small peptide inhibitors of α4β7 mediated MadCAM-1 adhesion to lymphocytes", Bio. Med, Chem. Lett., 1 2495 (1996); U.S Patent 5,510,332, PCT Publications W0 98/53814, W097/03094, W097/02289, W096/40781, W096/22966, W096/20216, W096/01644, W096106108, and W095/15973, and others.

Such small molecule agents may be produced by synthesizing a plurality of peptides (e.g., 5 to 20 amino acids in length), semi-peptidic compounds or non-peptidic, organic compounds, and then screening those compounds for their ability to inhibit the appropriate VLA-1/collagen or VLA-4/VCAM-1 interaction. See generally U.S. Patent No. 4,833,092, Scott and Smith, "Searching for Peptide Ligands with an Epitope Library", Science, 249, pp. 386-90 (1990), and Devlin et al., "Random Peptide Libraries: A Source of Specific Protein Binding Molecules", Science, 249, pp. 40407 (1990).

### B. Methods of Making Anti-Integrin Antibody Homologs

The technology for producing monoclonal antibodies, including for example, anti-integrin monoclonal antibodies is well known. See for example, Mendrick et al. 1995, *Lab. Invest.* 72:367-375 (mAbs to murine anti-α1β1 and anti-α2β1); Sonnenberg et al. 1987 *J. Biol. Chem.*262:10376-10383 (mAbs to murine anti-α6β1); Yao et al. 1996, *J Cell Sci* 1996 109:3139-50 (mAbs to murine anti-α7β1); Hemler et al. 1984, *J Immunol* 132:3011-8 (mAbs to human α1β1); Pischel et al. 1987 *J Immunol* 138:226-33 (mAbs to human α2β1); Wayner et al. 1988, *J Cell Biol* 107:1881-91 (mAbs to human α3β1); Hemler et al. 1987 *J Biol Chem* 262:11478-85 (mAbs to human α4β1); Wayner et al. 1988 *J Cell Biol* 107:1881-91 (mAbs to human α5β1); Sonnenberg et al. 1987, *J. Biol. Chem*. 262:10376-10383 (mAbs to human α6β1); A Wang et al. 1996 *Am. J. Respir. Cell Mol. Biol*. 15:664-672 (mAbs to human α9β1); Davies et al. 1989 *J Cell Biol* 109:1817-26 (mAbs to human αV β1); Sanchez-Madrid et al. 1982, *Proc Natl Acad Sci U S A* 79:7489-93 (mAbs to human αL β2); Diamond et al. 1993, *J Cell Biol* 120:1031-43 (mAbs to human αMβ2); Stacker et al. 1991 *J Immunol* 146:648-55 (mAbs to human αXβ2); Van der Vieren et al 1995 *Immunity* 3:683-90 (mAbs to human αDβ2); Bennett et al. 1983 *Proc Natl Acad Sci U S A* 80:2417-21 (mAbs to human αIIbβ3); Hessle et al. 1984, *Differentiation* 26:49-54 (mAbs to human α6β4); Weinacker et al. 1994 *J Biol Chem* 269:6940-8 (mAbs to human αVβ5); Weinacker et al. 1994 *J Biol Chem* 269:6940-8 (mAbs to human αVβ6); Cerf-Bensussan et al 1992 *Eur J Immunol* 22:273-7 (mAbs to human αEβ7); Nishimura et al. 1994 *J Biol Chem* 269:28708-15 (mAbs to human αVβ8); Bossy et al. 1991 *EMBO J* 10:2375-85 (polyclonal antisera to human α8β1); Camper et al. 1998 *J. Biol. Chem*. 273:20383-20389 (polyclonal antisera to human α10β1).

The preferred integrin antagonists contemplated herein can be expressed from intact or truncated genomic or cDNA or from synthetic DNAs in prokaryotic or eukaryotic host cells. The dimeric proteins can be isolated from the culture media and/or refolded and dimerized in vitro to form biologically active compositions. Heterodimers can be formed in vitro by combining separate, distinct polypeptide chains. Alternatively, heterodimers can be formed in a single cell by co-expressing nucleic acids encoding separate, distinct polypeptide chains. See, for example, WO93/09229, or U.S. Pat. No. 5,411,941, for several exemplary recombinant heterodimer protein production protocols. Currently preferred host cells include, without limitation, prokaryotes including E. coli, or eukaryotes including yeast, Saccharomyces, insect cells, or mammalian cells, such as CHO, COS or BSC cells. One of ordinary skill in the art will appreciate that other host cells can be used to advantage.

For example, anti-VLA-4 antibodies may be identified by immunoprecipitation of 125I-labeled cell lysates from VLA-4-expressing cells. (See, Sanchez-Madrid et al. 1986, Eur. J. Immunol., 16: 1343-1349 and Hemler et al. 1987, J. Biol. Chem., 262, 11478-11485). Anti-VLA-4 antibodies may also be identified by flow cytometry, e.g., by measuring fluorescent staining of Ramos cells incubated with an antibody believed to recognize VLA-4 (see, Elices et al., 1990 Cell, 60: 577-584). The lymphocytes used in the production of hybridoma cells typically are isolated from immunized mammals whose sera have already tested positive for the presence of anti-VLA-4 antibodies using such screening assays.

Typically, the immortal cell line (e.g., a myeloma cell line) is derived from the same mammalian species as the lymphocytes. Preferred immortal cell lines are mouse myeloma cell lines that are sensitive to culture medium containing hypoxanthine, aminopterin and thymidine ("HAT medium"). Typically, HAT-sensitive mouse myeloma cells are fused to mouse splenocytes using 1500 molecular weight polyethylene glycol ("PEG 1500"). Hybridoma cells resulting from the fusion are then selected using HAT medium, which kills unfused and unproductively fused myeloma cells (unfused splenocytes die after several days because they are not transformed). Hybridomas producing a desired antibody are detected by screening the hybridoma culture supernatants. For example, hybridomas prepared to produce anti-VLA-4 antibodies may be screened by testing the hybridoma culture supernatant for secreted antibodies having the ability to bind to a recombinant alpha4-subunit-expressing cell line (see, Elices et al., supra).

To produce anti-VLA-4 antibody homologs that are intact immunoglobulins, hybridoma cells that tested positive in such screening assays were cultured in a nutrient medium under conditions and for a time sufficient to allow the hybridoma cells to secrete the monoclonal antibodies into the culture medium. Tissue culture techniques and culture media suitable for hybridoma cells are well known. The conditioned hybridoma culture supernatant may be collected and the anti-VLA4 antibodies optionally further purified by well-known methods.

Alternatively, the desired antibody may be produced by injecting the hybridoma cells into the peritoneal cavity of an unimmunized mouse. The hybridoma cells proliferate in the peritoneal cavity, secreting the antibody which accumulates as ascites fluid. The antibody may be harvested by withdrawing the ascites fluid from the peritoneal cavity with a syringe.

Several mouse anti-VLA-4 monoclonal antibodies have been previously described. See, e.g., Sanchez-Madrid et al., 1986, supra; Hemler et al., 1987, supra; Pulido et al., 1991, J. Biol. Chem., 266 (16), 10241-10245); Issekutz and Wykretowicz, 1991, J. Immunol., 147: 109 (TA-2 mab). These anti-VLA-4 monoclonal antibodies and other anti-VLA-4 antibodies (e.g., U.S. Patent 5,888,507- Biogen, Inc. and references cited therein) capable of recognizing the alpha and/or beta chain of VLA-4 will be useful in the methods of treatment according to the present invention. AntiVLA-4 antibodies that will recognize the VLA-4 alpha4 chain epitopes involved in binding to VCAM-1 and fibronectin ligands (i.e., antibodies which can bind to VLA-4 at a site involved in ligand recognition and block VCAM-1 and fibronectin binding) are preferred. Such antibodies have been defined as B epitope-specific antibodies (B1 or B2) (Pulido et al., 1991, supra) and are also anti-VLA-4 antibodies according to the present invention.

Fully human monoclonal antibody homologs against VLA-4 are another preferred binding agent which may block or coat VLA-4 ligands in the method of the invention. In their intact form these may be prepared using in vitro-primed human splenocytes, as described by Boerner et al., 1991, J. Immunol., 147, 86-95. Alternatively, they may be prepared by repertoire cloning as described by Persson et al., 1991, Proc. Nat. Acad. Sci. USA, 88: 2432-2436 or by Huang and Stollar, 1991, J. Immunol. Methods 141, 227-236. U.S. Patent 5,798,230 (Aug. 25, 1998, "Process for the preparation of human monoclonal antibodies and their use") who describe preparation of human monoclonal antibodies from human B cells. According to this process, human antibody-producing B cells are immortalized by infection with an Epstein-Barr virus, or a derivative thereof, that expresses Epstein-Barr virus nuclear antigen 2 (EBNA2). EBNA2 function, which is required for immortalization, is subsequently shut off, which results in an increase in antibody production.

In yet another method for producing fully human antibodies, United States Patent 5,789,650 (Aug. 4, 1998, "Transgenic non-human animals for producing heterologous antibodies") describes transgenic non-human animals capable of producing heterologous antibodies and transgenic non-human animals having inactivated endogenous immunoglobulin genes. Endogenous immunoglobulin genes are suppressed by antisense polynucleotides and/or by antiserum directed against endogenous immunoglobulins. Heterologous antibodies are encoded by immunoglobulin genes not normally found in the genome of that species of non-human animal. One or more transgenes containing sequences of unrearranged heterologous human immunoglobulin heavy chains are introduced into a non-human animal thereby forming a transgenic animal capable of functionally rearranging transgenic immunoglobulin sequences and producing a repertoire of antibodies of various isotypes encoded by human immunoglobulin genes. Such heterologous human antibodies are produced in B-cells which are thereafter immortalized, e.g., by fusing with an immortalizing cell line such as a myeloma or by manipulating such B-cells by other techniques to perpetuate a cell line capable of producing a monoclonal heterologous, fully human antibody homolog.

Large nonimmunized human phage display libraries may also be used to isolate high affinity antibodies that can be developed as human therapeutics using standard phage technology (Vaughan et al, 1996).

Yet another preferred binding agent which may block or coat integrin ligands in the method of the invention is a humanized recombinant antibody homolog having anti-integrin specificity. Following the early methods for the preparation of true "chimeric antibodies" (where the entire constant and entire variable regions are derived from different sources), a new approach was described in EP 0239400 (Winter et al.) whereby antibodies are altered by substitution (within a given variable region) of their complementarity determining regions (CDRs) for one species with those from another. This process may be used, for example, to substitute the CDRs from human heavy and light chain Ig variable region domains with alternative CDRs from murine variable region domains. These altered Ig variable regions may subsequently be combined with human Ig constant regions to created antibodies which are totally human in composition except for the substituted murine CDRs. Such CDR-substituted antibodies would be predicted to be less likely to elicit an immune response in humans compared to true chimeric antibodies because the CDR-substituted antibodies contain considerably less non-human components. The process for humanizing monoclonal antibodies via CDR "grafting" has been termed "reshaping". (Riechmann et al., 1988, Nature 332, 323-327; Verhoeyen et al., 1988, Science 239, 1534-1536).

Typically, complementarity determining regions (CDRs) of a murine antibody are transplanted onto the corresponding regions in a human antibody, since it is the CDRs (three in antibody heavy chains, three in light chains) that are the regions of the mouse antibody which bind to a specific antigen. Transplantation of CDRs is achieved by genetic engineering whereby CDR DNA sequences are determined by cloning of murine heavy and light chain variable (V) region gene segments, and are then transferred to corresponding human V regions by site directed mutagenesis. In the final stage of the process, human constant region gene segments of the desired isotype (usually gamma I for CH and kappa for CL) are added and the humanized heavy and light chain genes are co-expressed in mammalian cells to produce soluble humanized antibody.

The transfer of these CDRs to a human antibody confers on this antibody the antigen binding properties of the original murine antibody. The six CDRs in the murine antibody are mounted structurally on a V region "framework" region. The reason that CDR-grafting is successful is that framework regions between mouse and human antibodies may have very similar 3-D structures with similar points of attachment for CDRS, such that CDRs can be interchanged. Such humanized antibody homologs may be prepared, as exemplified in Jones et al., 1986, Nature 321, 522-525; Riechmann, 1988, Nature 332, 323-327; Queen et al., 1989, Proc. Nat. Acad. Sci. USA 86, 10029; and Orlandi et al., 1989, Proc. Nat. Acad. Sci. USA 86,3833.

Nonetheless, certain amino acids within framework regions are thought to interact with CDRs and to influence overall antigen binding affinity. The direct transfer of CDRs from a murine antibody to produce a recombinant humanized antibody without any modifications of the human V region frameworks often results in a partial or complete loss of binding affinity. In a number of cases, it appears to be critical to alter residues in the framework regions of the acceptor antibody in order to obtain binding activity.

Queen et al., 1989 (supra) and WO 90/07861 (Protein Design Labs) have described the preparation of a humanized antibody that contains modified residues in the framework regions of the acceptor antibody by combining the CDRs of a murine MAb (anti-Tac) with human immunoglobulin framework and constant regions. They have demonstrated one solution to the problem of the loss of binding affinity that often results from direct CDR transfer without any modifications of the human V region framework residues; their solution involves two key steps. First, the human V framework regions are chosen by computer analysts for optimal protein sequence homology to the V region framework of the original murine antibody, in this case, the anti-Tac MAb. In the second step, the tertiary structure of the murine V region is modelled by computer in order to visualize framework amino acid residues which are likely to interact with the murine CDRs and these murine amino acid residues are then superimposed on the homologous human framework. See also U.S. Patents 5,693,762; 5,693,761; 5,585,089; and 5,530,101 (Protein Design Labs).

One may use a different approach (Tempest et al., 1991, Biotechnology 9, 266-271) and utilize, as standard, the V region frameworks derived from NEWM and REI heavy and light chains respectively for CDR-grafting without radical introduction of mouse residues. An advantage of using the Tempest et al., approach to construct NEWM and REI based humanized antibodies is that the 3dimensional structures of NEWM and REI variable regions are known from x-ray crystallography and thus specific interactions between CDRs and V region framework residues can be modeled.

Regardless of the approach taken, the examples of the initial humanized antibody homologs prepared to date have shown that it is not a straightforward process. However, even acknowledging that such framework changes may be necessary, it is not possible to predict, on the basis of the available prior art, which, if any, framework residues will need to be altered to obtain functional humanized recombinant antibodies of the desired specificity. Results thus far indicate that changes necessary to preserve specificity and/or affinity are for the most part unique to a given antibody and cannot be predicted based on the humanization of a different antibody.

Certain alpha4 subunit-containing integrin antagonists useful in the present invention include chimeric and humanized recombinant antibody homologs ( i.e., intact immunoglobulins and portions thereof) with B epitope specificity that have been prepared and are described in U.S. Patent 5,932,214( mab HP1/2). The starting material for the preparation of chimeric (mouse Variable - human Constant) and humanized anti-integrin antibody homologs may be a murine monoclonal anti-integrin antibody as previously described, a monoclonal anti-integrin antibody commercially available (e.g., HP2/1, Amae International, Inc., Westbrook, Maine), or a monoclonal anti-integrin antibody prepared in accordance with the teaching herein. Other preferred humanized anti-VLA4 antibody homologs are described by Athena Neurosciences, Inc. in PCT/US95/01219 (27 July 1995) and U.S. Patent 5,840,299.

These humanized anti-VLA-4 antibodies comprise a humanized light chain and a humanized heavy chain. The humanized light chain comprises three complementarity determining regions (CDRI, CDR2 and CDR3) having amino acid sequences from the corresponding complementarity determining regions of a mouse 21- 6 immunoglobulin light chain, and a variable region framework from a human kappa light chain variable region framework sequence except in at least position the amino acid position is occupied by the same amino acid present in the equivalent position of the mouse 21.6 immunoglobulin light chain variable region framework. The humanized heavy chain comprises three complementarity determining regions (CDR1, CDR2 and CDR3) having amino acid sequences from the corresponding complementarity determining regions of a mouse 21-6 immunoglobulin heavy chain, and a variable region framework from a human heavy chain variable region framework sequence except in at least one position the amino acid position is occupied by the same amino acid present in the equivalent position of the mouse 21-6 immunoglobulin heavy chain variable region framework.

The present invention may utilize antagonists encoded by nucleic acid sequences that hybridize under stringent conditions to nucleic acid sequences encoding antibodies directed against alpha4 subunit containing integrins. For example, an antagonist of the present invention may be a protein whose nucleic acid hybridizes under high stringency conditions to one or more of those nucleic acid sequence found in Table 6 of U.S. Patent 5,840,299 or the complement of such one or more sequences. Antagonists may also be a protein whose nucleic acid hybridizes under high stringency conditions to a nucleic acid encoding SEQ ID NO: 2 or SEQ ID NO: 4 found in U.S. Patent 5,932,214. Further, antagonists may also be a protein whose nucleic acid hybridizes under high stringency conditions to a nucleic acid encoding a variable domain of the antibody produced by cell line ATCC CRL 11175.

Alternatively, an antagonist of the present invention may be a protein whose nucleic acid hybridizes under low stringency conditions to one or more of those nucleic acid sequence found in Table 6 of U.S. Patent 5,840,299 or the complement of such one or more sequences. Antagonists may also be a protein whose nucleic acid hybridizes under low stringency conditions to a nucleic acid encoding SEQ ID NO: 2 or SEQ ID NO: 4 found in U.S. Patent 5,932,214. Further, antagonists may also be a protein whose nucleic acid hybridizes under low stringency conditions to a nucleic acid encoding a variable domain of the antibody produced by cell line ATCC CRL 11175.

### C. Production of Fragments and Analogs

Fragments of an isolated alpha4 integrin antagonists (e.g., fragments of antibody homologs described herein) can also be produced efficiently by recombinant methods, by proteolytic digestion, or by chemical synthesis using methods known to those of skill in the art. In recombinant methods, internal or terminal fragments of a polypeptide can be generated by removing one or more nucleotides from one end (for a terminal fragment) or both ends (for an internal fragment) of a DNA sequence which encodes for the isolated hedgehog polypeptide. Expression of the mutagenized DNA produces polypeptide fragments. Digestion with "end nibbling" endonucleases can also generate DNAs which encode an array of fragments. DNAs which encode fragments of a protein can also be generated by random shearing, restriction digestion, or a combination or both. Protein fragments can be generated directly from intact proteins. Peptides can be cleaved specifically by proteolytic enzymes, including, but not limited to plasmin, thrombin, trypsin, chymotrypsin, or pepsin. Each of these enzymes is specific for the type of peptide bond it attacks. Trypsin catalyzes the hydrolysis of peptide bonds in which the carbonyl group is from a basic amino acid, usually arginine or lysine. Pepsin and chymotrypsin catalyse the hydrolysis of peptide bonds from aromatic amino acids, such as tryptophan, tyrosine, and phenylalanine. Alternative sets of cleaved protein fragments are generated by preventing cleavage at a site which is suceptible to a proteolytic enzyme. For instance, reaction of the ε-amino acid group of lysine with ethyltrifluorothioacetate in mildly basic solution yields blocked amino acid residues whose adjacent peptide bond is no longer susceptible to hydrolysis by trypsin. Proteins can be modified to create peptide linkages that are susceptible to proteolytic enzymes. For instance, alkylation of cysteine residues with β-haloethylamines yields peptide linkages that are hydrolyzed by trypsin (Lindley, (1956) Nature 178, 647). In addition, chemical reagents that cleave peptide chains at specific residues can be used. For example, cyanogen bromide cleaves peptides at methionine residues (Gross and Witkip, (1961) J. Am. Chem. Soc. 83, 1510). Thus, by treating proteins with various combinations of modifiers, proteolytic enzymes and/or chemical reagents, the proteins may be divided into fragments of a desired length with no overlap of the fragments, or divided into overlapping fragments of a desired length.

Fragments can also be synthesized chemically using techniques known in the art such as the Merrifield solid phase F moc or t-Boc chemistry. **Merrifield, Recent Progress in Hormone Research 23: 451 (1967)**

Examples of prior art methods which allow production and testing of fragments and analogs are discussed below. These, or analogous methods may be used to make and screen fragments and analogs of an isolated alpha4 integrin antagonist which can be shown to have biological activity. An exemplary method to test whether fragments and analogs of alpha 4 subunit containing integrin antagonists have biological activity is found in Section IV and the Examples.

### D. Production of Altered DNA and Peptide Sequences: Random Methods

Amino acid sequence variants of a protein can be prepared by random mutagenesis of DNA which encodes the protein or a particular portion thereof. Useful methods include PCR mutagenesis and saturation mutagenesis. A library of random amino acid sequence variants can also be generated by the synthesis of a set of degenerate oligonucleotide sequences. Methods of generating amino acid sequence variants of a given protein using altered DNA and peptides are well-known in the art. The following examples of such methods are not intended to limit the scope of the present invention, but merely serve to illustrate representative techniques. Persons having ordinary skill in the art will recognize that other methods are also useful in this regard.
PCR Mutagenesis: See, for example Leung et al., (1989) Technique 1, 11-15.
Saturation Mutagenesis: One method is described generally in Mayers et al., (1989) Science 229, 242.
Degenerate Oligonucleotide Mutagenesis: See for example Harang, S.A., (1983) Tetrahedron 39, 3; Itakura et al., (1984) Ann. Rev. Biochem. 53, 323 and Itakura et al., Recombinant DNA, Proc. 3rd Cleveland Symposium on Macromolecules, pp. 273-289 (A.G. Walton, ed.), Elsevier, Amsterdam, 1981.

### E. Production of Altered DNA and Peptide Sequences: Directed Methods

Non-random, or directed, mutagenesis provides specific sequences or mutations in specific portions of a polynucleotide sequence that encodes an isolated polypeptide, to provide variants which include deletions, insertions, or substitutions of residues of the known amino acid sequence of the isolated polypeptide. The mutation sites may be modified individually or in series, for instance by: (1) substituting first with conserved amino acids and then with more radical choices depending on the results achieved; (2) deleting the target residue; or (3) inserting residues of the same or a different class adjacent to the located site, or combinations of options 1-3.

Clearly, such site-directed methods are one way in which an N-terminal cysteine (or a functional equivalent) can be introduced into a given polypeptide sequence to provide the attachment site for a hydrophobic moiety.
Alanine scanning Mutagenesis: See Cunningham and Wells, (1989) Science 244, 1081-1085).
Oligonucleotide-Mediated Mutagenesis: See, for example, Adelman et al., (1983) DNA 2, 183.
Cassette Mutagenesis: See Wells et al., (1985) Gene 34, 315.
Combinatorial Mutagenesis: See, for example, Ladner et al., WO 88/06630
Phage Display Strategies: See, for example the review by Marks et al., J. Biol. Chemistry: 267 16007-16010 (1992).

### F. Other Variants of Integrin antagonists

Variants can differ from other integrin antagonists described herein in amino acid sequence or in ways that do not involve sequence, or both. The most preferred polypeptides of the invention have preferred non-sequence modifications that include *in vivo* or *in vitro* chemical derivatization (e.g., of their N-terminal end), as well as possible changes in acetylation, methylation, phosphorylation, amidation, carboxylation, or glycosylation.

Other analogs include a protein or its biologically active fragments whose sequences differ from those found in U.S. Patents 5,840,299 or U.S. 5,888,507; U.S. 5,932,214 or PCT US/94/00266 by one or more conservative amino acid substitutions or by one or more non conservative amino acid substitutions, or by deletions or insertions which do not abolish the isolated protein's biological activity. Conservative substitutions typically include the substitution of one amino acid for another with similar characteristics such as substitutions within the following groups: valine, alanine and glycine; leucine and isoleucine; aspartic acid and glutamic acid; asparagine and glutamine; serine and threonine; lysine and arginine; and phenylalanine and tyrosine. The non-polar hydrophobic amino acids include alanine, leucine, isoleucine, valine, proline, phenylalanine, tryptophan, and methionine. The polar neutral amino acids include glycine, serine, threonine, cysteine, tyrosine, asparagine, and glutamine. The positively charged (basic) amino acids include arginine, lysine, and histidine. The negatively charged (acidic) amino acids include aspartic acid and glutamic acid. Other conservative substitutions can be readily known by workers of ordinary skill. For example, for the amino acid alanine, a conservative substitution can be taken from any one of D-alanine, glycine, beta-alanine, L-cysteine, and D-cysteine. For lysine, a replacement can be any one of D-lysine, arginine, D-arginine, homo-arginine, methionine, D-methionine, ornithine, or D-ornithine.

Other analogs used within the invention are those with modifications which increase peptide stability. Such analogs may contain, for example, one or more non-peptide bonds (which replace the peptide bonds) in the peptide sequence. Also included are: analogs that include residues other than naturally occurring L-amino acids, such as D-amino acids or non-naturally occurring or synthetic amino acids such as beta or gamma amino acids and cyclic analogs. Incorporation of D- instead of L-amino acids into the isolated hedgehog polypeptide may increase its resistance to proteases. See, U.S. Patent 5,219,990 *supra.*

Preferred antibody homologs include an amino acid sequence at least 60%, 80%, 90%, 95%, 98%, or 99% homologous to an amino acid sequence of PS/2 antibody (See Example) or include an amino acid sequence at least 60%, 80%, 90%, 95%, 98%, or 99% homologous to an amino acid sequences described in U.S. Patent 5,840,299 (SEQ ID NO 15-light chain variable region or SEQ ID NO: 17- heavy chain variable region) or U.S. Patent 5,932,214 (SEQ ID NOS: 2 or 4); and published patent application W094/16094 (those sequences found in the anti-VLA4 antibody of deposited cell line ATCC CRL 11175).

### G. Polymer Conjugate Forms

Within the broad scope of the present invention, a single polymer molecule may be employed for conjugation with an alpha 1 or alpha4 integrin antagonist, although it is also contemplated that more than one polymer molecule can be attached as well. Conjugated alpha4 integrin antagonist compositions of the invention may find utility in both *in vivo* as well as non-*in vivo* applications. Additionally, it will be recognized that the conjugating polymer may utilize any other groups, moieties, or other conjugated species, as appropriate to the end use application. By way of example, it may be useful in some applications to covalently bond to the polymer a functional moiety imparting UV-degradation resistance, or antioxidation, or other properties or characteristics to the polymer. As a further example, it may be advantageous in some applications to functionalize the polymer to render it reactive and enable it to cross-link to a drug molecule, to enhance various properties or characteristics of the overall conjugated material. Accordingly, the polymer may contain any functionality, repeating groups, linkages, or other constitutent structures which do not preclude the efficacy of the conjugated alpha4 integrin antagonist composition for its intended purpose. Other objectives and advantages of the present invention will be more fully apparent from the ensuing disclosure and appended claims.

Illustrative polymers that may usefully be employed to achieve these desirable characteristics are described herein below in exemplary reaction schemes. In covalently bonded antagonist/polymer conjugates, the polymer may be functionalized and then coupled to free amino acid(s) of the antagonist to form labile bonds.

Antagonists to alpha4 or alpha1 subunit containing integrins are conjugated most preferably via a terminal reactive group on the polymer although conjugations can also be branched from non-terminal reactive groups. The polymer with the reactive group(s) is designated herein as "activated polymer". The reactive group selectively reacts with free amino or other reactive groups on the antagonist molecule. The activated polymer(s) is reacted so that attachment may occur at any available alpha4 integrin antagonist amino group such as the alpha amino groups or the epsilon-amino groups of lysines. Free carboxylic groups, suitably activated carbonyl groups, hydroxyl, guanidyl, oxidized carbohydrate moieties and mercapto groups of the alpha4 integrin antagonist (if available) can also be used as attachment sites.

Although the polymer may be attached anywhere on the integrin antagonist molecule, a preferred site for polymer coupling to integrin antagonists (particularly those that are proteins) is the N-terminus of the integrin antagonist. Secondary site(s) are at or near the C-terminus and through sugar moieties (if any). Thus, the invention contemplates: (i) N-terminally coupled polymer conjugates of alphal and alpha4 integrin antagonists; (ii) C-terminally coupled polymer conjugates of alphal and alpha4 integrin antagonists; (iii) sugar-coupled conjugates; (iv) as well as N-, C- and sugar-coupled polymer conjugates of alphal and alpha4 integrin antagonists.

Generally from about 1.0 to about 10 moles of activated polymer per mole of antagonist, depending on antagonist concentration, is employed. The final amount is a balance between maximizing the extent of the reaction while minimizing non-specific modifications of the product and, at the same time, defining chemistries that will maintain optimum activity, while at the same time optimizing, if possible, the half-life of the antagonist. Preferably, at least about 50% of the biological activity of the antagonist is retained, and most preferably 100% is retained.

The reactions may take place by any suitable art-recognized method used for reacting biologically active materials with inert polymers. Generally the process involves preparing an activated polymer (that may have at least one terminal hydroxyl group) and thereafter reacting the antagonist with the activated polymer to produce the soluble protein suitable for formulation. The above modification reaction can be performed by several methods, which may involve one or more steps.

As mentioned above, certain embodiments of the invention utilize the N-terminal end of an integrin antagonist as the linkage to the polymer. Suitable conventional methods are available to selectively obtain an N-terminally modified alpha1 or alpha4 integrin antagonist. One method is exemplified by a reductive alkylation method which exploits differential reactivity of different types of primary amino groups (the epsilon amino groups on the lysine versus the amino groups on an N-terminal methionine) available for derivatization on a suitable integrin antagonist. Under the appropriate selection conditions, substantially selective derivatization of a suitable integrin antagonist at an N-terminus thereof with a carbonyl group containing polymer can be achieved. The reaction is performed at a pH which allows one to take advantage of the pKa differences between the epsilon-amino groups of the lysine residues and that of the alpha-amino group of an N-terminal residue of the integrin antagonist. This type of chemistry is well known to persons with ordinary skill in the art.

A strategy for targeting a polyalkylene glycol polymer such as PEG to the C-terminus of an alpha1 or alpha4 integrin antagonist (e.g., as a protein) would be to chemically attach or genetically engineer a site that can be used to target the polymer moiety. For example, incorporation of a Cys at a site that is at or near the C-terminus of a protein would allow specific modification using art recognized maleimide, vinylsulfone or haloacetate- activated derivatives of polyalkylene glycol (e.g., PEG). These derivatives can be used specifically for modification of the engineered cysteines due to the high selectively of these reagents for Cys. Other strategies such as incorporation of a histidine tag which can be targeted (Fancy et al., (1996) Chem. & Biol. 3: 551) or an additional glycosylation site on a protein, represent other alternatives for modifying the C-terminus of an integrin antagonist of the invention.

Methods for targeting sugars as sites for chemical modification are also well known and therefore it is likely that a polyalkylene glycol polymer can be added directly and specifically to sugars (if any) on an integrin antagonist that have been activated through oxidation. For example, a polyethyleneglycol-hydrazide can be generated which forms relatively stable hydrazone linkages by condensation with aldehydes and ketones. This property has been used for modification of proteins through oxidized oligosaccharide linkages. See Andresz, H. et al., (1978), Makromol. Chem. 179: 301. In particular, treatment of PEG-carboxymethyl hydrazide with nitrite produces PEG-carboxymethyl azide which is an electrophilically active group reactive toward amino groups. This reaction can be used to prepare polyalkylene glycol-modified proteins as well. See, U.S. Patents 4,101,380 and 4,179,337.

One can use art recognized thiol linker-mediated chemistry to further facilitate cross-linking of proteins to form multivalent alpha1 or alpha 4 integrin antagonist compositions. In particular, one can generate reactive aldehydes on carbohydrate moieties with sodium periodate, forming cystamine conjugates through the aldehydes and inducing cross-linking via the thiol groups on the cystamines. See Pepinsky, B. et al., (1991), J. Biol. Chem., 266: 18244-18249 and Chen, L.L. et al., (1991) J. Biol. Chem., 266: 18237-18243. Therefore, this type of chemistry would also be appropriate for modification with polyalkylene glycol polymers where a linker is incorporated into the sugar and the polyalkylene glycol polymer is attached to the linker. While aminothiol or hydrazine-containing linkers will allow for addition of a single polymer group, the structure of the linker can be varied so that multiple polymers are added and/or that the spatial orientation of the polymer with respect to the integrin antagonist is changed.

In the practice of the present invention, polyalkylene glycol residues of C1-C4 alkyl polyalkylene glycols, preferably polyethylene glycol (PEG), or poly(oxy)alkylene glycol residues of such glycols are advantageously incorporated in the polymer systems of interest. Thus, the polymer to which the protein is attached can be a homopolymer of polyethylene glycol (PEG) or is a polyoxyethylated polyol, provided in all cases that the polymer is soluble in water at room temperature. Non-limiting examples of such polymers include polyalkylene oxide homopolymers such as PEG or polypropylene glycols, polyoxyethylenated glycols, copolymers thereof and block copolymers thereof, provided that the water solubility of the block copolymer is maintained. Examples of polyoxyethylated polyols include, for example, polyoxyethylated glycerol, polyoxyethylated sorbitol, polyoxyethylated glucose, or the like. The glycerol backbone of polyoxyethylated glycerol is the same backbone occurring naturally in, for example, animals and humans in mono-, di-, and triglycerides. Therefore, this branching would not necessarily be seen as a foreign agent in the body.

As an alternative to polyalkylene oxides, dextran, polyvinyl pyrrolidones, polyacrylamides, polyvinyl alcohols, carbohydrate-based polymers and the like may be used. Those of ordinary skill in the art will recognize that the foregoing list is merely illustrative and that all polymer materials having the qualities described herein are contemplated.

The polymer need not have any particular molecular weight, but it is preferred that the molecular weight be between about 300 and 100,000, more preferably between 10,000 and 40,000. In particular, sizes of 20,000 or more are best at preventing loss of the product due to filtration in the kidneys.

Polyalkylene glycol derivatization has a number of advantageous properties in the formulation of polymer-integrin antagonist conjugates in the practice of the present invention, as associated with the following properties of polyalkylene glycol derivatives: improvement of aqueous solubility, while at the same time eliciting no antigenic or immunogenic response; high degrees of biocompatibility; absence of in vivo biodegradation of the polyalkylene glycol derivatives; and ease of excretion by living organisms.

Moreover, in another aspect of the invention, one can utilize an alpha1 or alpha4 integrin antagonist covalently bonded to the polymer component in which the nature of the conjugation involves cleavable covalent chemical bonds. This allows for control in terms of the time course over which the polymer may be cleaved from the integrin antagonist. This covalent bond between the integrin antagonist and the polymer may be cleaved by chemical or enzymatic reaction. The polymer-integrin antagonist product retains an acceptable amount of activity. Concurrently, portions of polyethylene glycol are present in the conjugating polymer to endow the polymer-integrin antagonist conjugate with high aqueous solubility and prolonged blood circulation capability. As a result of these improved characteristics the invention contemplates parenteral, nasal, and oral delivery of both the active polymer-alpha4 integrin antagonist species and, following hydrolytic cleavage, bioavailability of the integrin antagonist per se, in in vivo applications.

It is to be understood that the reaction schemes described herein are provided for the purposes of illustration only and are not to be limiting with respect to the reactions and structures which may be utilized in the modification of the alpha1 or alpha4 integrin antagonist, e.g., to achieve solubility, stabilization, and cell membrane affinity for parenteral and oral administration. The activity and stability of these integrin antagonist conjugates can be varied in several ways, by using a polymer of different molecular size. Solubilities of the conjugates can be varied by changing the proportion and size of the polyethylene glycol fragment incorporated in the polymer composition.

### III. Utilities

The amount of active ingredient that may be combined with the carrier materials to produce a single dosage form will vary depending upon the subject treated, and the particular mode of administration. It should be understood, however, that a specific dosage and treatment regimen for any particular subject will depend upon a variety of factors, including the activity of the specific compound employed, the age, body weight, general health, sex, diet, time of administration, rate of excretion, drug combination, and the judgment of the treating physician and the severity of the particular disease being treated. The amount of active ingredient may also depend upon the therapeutic or prophylactic agent, if any, with which the ingredient is co-administered.

According to this invention, the antagonists of the present invention are to be administered to the subject in an effective amount. Doses in the inventive use are an efficacious, non toxic quantity. Persons skilled in the art of using routine clinical testing are able to determine optimum doses for the particular ailment being treated.

### Pharmaceutical Preparations

According to the invention, antagonists of the invention may be administered parenterally. The term "parenteral" as used herein includes subcutaneous, intravenous, intrarnuscular, intra-articular, intra-synovial, intrasternal, intrathecal, intrahepatic, intralesional and intracranial injection or infusion techniques. The desired dose is to be administered to a subject one or more times daily, intravenously, orally, rectally, parenterally, intranasally, topically, or by inhalation. The desired dose may also be given by continuous intravenous infusion.

Antibody homologs are preferably administered as a sterile pharmaceutical composition containing a pharmaceutically acceptable carrier, which may be any of the numerous well known carriers, such as water, saline, phosphate buffered saline, dextrose, glycerol, ethanol, and the like, or combinations thereof. The compounds of the present invention may be used in the form of pharmaceutically acceptable salts derived from inorganic or organic acids and bases. Included among such acid salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate and undecanoate. Base salts include ammonium salts, alkali metal salts, such as sodium and potassium salts, alkaline earth metal salts, such as calcium and magnesium salts, salts with organic bases, such as dicyclohexylamine salts, N-methyl-D-glucamine, tris(hydroxymethyl)methylamine and salts with amino acids such as arginine, lysine, and so forth. Also, the basic nitrogen-containing groups can be quatemized with such agents as lower alkyl halides, such as methyl, ethyl, propyl, and butyl chloride, bromides and iodides; dialkyl sulfates, such as dimethyl, diethyl, dibutyl and diamyl sulfates, long chain halides such as decyl, lauryl, myristyl and stearyl chlorides, bromides and iodides, aralkyl halides, such as benzyl and phenethyl bromides and others. Water or oil-soluble or dispersible products are thereby obtained.

The pharmaceutical compositions of this invention comprise any of the compounds of the present invention, or pharmaceutically acceptable derivatives thereof, together with any pharmaceutically acceptable carrier. The term "carrier" as used herein includes acceptable adjuvants and vehicles. Pharmaceutically acceptable carriers that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

According to this invention, the pharmaceutical compositions may be in the form of a sterile injectable preparation, for example a sterile injectable aqueous or oleaginous suspension. This suspension may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as do natural pharmaceutically-acceptable oils, such as olive oil or castor oil, especially in their polyoxyethylated versions.

The pharmaceutical compositions of this invention may be given orally. If given orally, they can be administered in any orally acceptable dosage form including, but not limited to, capsules, tablets, aqueous suspensions or solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added.

Particular compositions for use in the present invention are those wherein the antagonist is formulated in vesicles such as liposome-containing compositions. Liposomes are vesicles formed by amphiphatic molecules such as polar lipids, for example, phosphatidyl cholines, ethanolamines and serines, sphingomyelins, cardiolipins, plasmalogens, phosphatidic acids and cerebiosides. Liposomes are formed when suitable amphiphathic molecules are allowed to swell in water or aqueous solutions to form liquid crystals usually of multilayer structure comprised of many bilayers separated from each other by aqueous material (also referred to as coarse liposomes). Another type of liposome known to be consisting of a single bilayer encapsulating aqueous material is referred to as a unilamellar vesicle. If watersoluble materials are included in the aqueous phase during the swelling of the lipids they become entrapped in the aqueous layer between the lipid bilayers.

A particularly convenient method for preparing liposome formulated forms of the present antagonists is the method described in EP-A-253,619.

In this method, single bilayered liposomes containing encapsulated active ingredients are prepared by dissolving the lipid component in an organic medium, injecting the organic solution of the lipid component under pressure into an aqueous component while simultaneously mixing the organic and aqueous components with a high speed homogenizer or mixing means, whereupon the liposomes are formed spontaneously. The single bilayered liposomes containing the encapsulated active ingredient can be employed directly or they can be employed in a suitable pharmaceutically acceptable carrier for topical administration. The viscosity of the liposomes can be increased by the addition of one or more suitable thickening agents such as, for example xanthan gum, hydroxypropyl cellulose, hydroxypropyl methylcellulose and mixtures thereof. The aqueous component may consist of water alone or it may contain electrolytes, buffered systems and other ingredients, such as, for example, preservatives. Suitable electrolytes which can be employed include metal salts such as alkali metal and alkaline earth metal salts. The preferred metal salts are calcium chloride, sodium chloride and potassium chloride. The concentration of the electrolyte may vary from zero to 260 mM, preferably from 5 mM to 160 mM. The aqueous component is placed in a suitable vessel which can be adapted to effect homogenization by effecting great turbulence during the injection of the organic component. Homogenization of the two components can be accomplished within the vessel, or, alternatively, the aqueous and organic components may be injected separately into a mixing means which is located outside the vessel. In the latter case, the liposomes are formed in the mixing means and then transferred to another vessel for collection purpose.

The organic component consists of a suitable non-toxic, pharmaceutically acceptable solvent such as, for example ethanol, glycerol, propylene glycol and polyethylene glycol, and a suitable phospholipid which is soluble in the solvent. Suitable phospholipids which can be employed include lecithin, phosphatidylcholine, phosphatydylserine, phosphatidylethanol-amine, phosphatidylinositol, lysophosphatidylcholine and phospha-tidyl glycerol, for example. Other lipophilic additives may be employed in order to selectively modify the characteristics of the liposomes. Examples of such other additives include stearylamine, phosphatidic acid, tocopherol, cholesterol and lanolin extracts.

In addition, other ingredients which can prevent oxidation of the phospholipids may be added to the organic component. Examples of such other ingredients include tocopherol, butylated hydroxyanisole, butylated hydroxytoluene, ascorbyl palmitate and ascorbyl oleate. Preservatives such a benzoic acid, methyl paraben and propyl paraben may also be added.

Apart from the above-described compositions, use may be made of covers, e.g. plasters, bandages, dressings, gauze pads and the like, containing an appropriate amount of an anti-VLA antibody therapeutic. In some cases use may be made of plasters, bandages, dressings, gauze pads and the like which have been impregnated with a topical formulation containing the therapeutic formulation.

The pharmaceutical compositions of this invention may also be administered by nasal aerosol or inhalation through the use of a nebulizer, a dry powder inhaler or a metered dose inhaler. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

According to another embodiment compositions containing a compound of this invention may also comprise an additional agent selected from the group consisting of corticosteroids, antiinflammatories, immunosuppressants, antimetabolites, and immunomodulators. Specific compounds within each of these classes may be selected from any of those listed under the appropriate group headings in "Comprehensive Medicinal Chemistry", Pergamon Press, Oxford, England, pp. 970-986 (1990), the disclosure of which is herein incorporated by reference. Also included within this group are compounds such as theophylline, sulfasalazine and aminosalicylates (antiinflammatories); cyclosporin, FK-506, and rapamycin (immunosuppressants); cyclophosphamide and methotrexate (antimetabolites); steroids (inhaled , oral or topical) and interferons (immunomodulators).

The dosage and dose rate of the compounds of this invention effective to produce the desired effects will depend on a variety of factors, such as the nature of the antagonist, the size of the subject, the goal of the treatment, the nature of the pathology to be treated, the specific pharmaceutical composition used, and the judgment of the treating physician.

Dosage levels of between about 0.001 and about 100 mg/kg body weight per day, preferably between about 0.1 and about 50 mg/kg body weight per day of the active ingredient compound are useful. Most preferably, the VLA-4 binding agent, if an antibody or antibody derivative, will be administered at a dose ranging between about 0.1 mg/kg body weight/day and about 20 mg/kg body weight/day, preferably ranging between about 0.1 mg/kg body weight/day and about 10 mg/kg body weight/day and at intervals of every 1-14 days. For non-antibody or small molecule binding agents; the dose range should preferably be between molar equivalent amounts to these amounts of antibody. Preferably, an antibody composition is administered in an amount effective to provide a plasma level of antibody of at least 1 mg/ml. Optimization of dosages can be determined by administration of the binding agents, followed by assessment of the coating of integrin-positive cells by the agent over time after administered at a given dose in vivo.

The presence of the administered agent may be detected in vitro (or ex vivo) by the inability or decreased ability of the individual's cells to bind the same agent which has been itself labelled (e.g., by a fluorochrome). The preferred dosage should produce detectable coating of the vast majority of integrin-positive cells. Preferably, coating is sustained in the case of an antibody homolog for a 1- 14 day period.

Persons having ordinary skill in the art can readily test if an antagonist of the invention is having it intended effect. Standard tests for clinical recovery (e.g. lavage and FACS scan for antibody binding; improvement in forced vital lung capacity) will be employed by skilled artisans to determine efficacy. For instance, cells contained in a sample of the individual's lung tissue are probed for the presence of the agent in vitro (or ex vivo) using a second reagent to detect the administered agent. For example, this may be a fluorochrome labelled antibody specific for the administered agent which is then measured by standard FACS (fluorescence activated cell sorter) analysis. Alternatively, presence of the administered agent is detected in vitro (or ex vivo) by the inability or decreased ability of the individual's cells to bind the same agent which has been itself labelled (e.g., by a fluorochrome). The preferred dosage should produce detectable coating of the vast majority of integrin-positive cells. Preferably, coating is sustained in the case of an antibody homolog for a 1- 14 day period.

The following Examples are provided to illustrate the present invention, and should not be construed as limiting thereof.

### EXAMPLE I: PULMONARY FIBROSIS ANIMAL MODELS

Much evidence has documented the involvement of inflammatory cells and mediators in pulmonary fibrosis in widely used bleomycin (BL)-rodent models. This model is appealing because it produces a characteristic picture of fibrosis with many of the components of human disease, and because BL-induced pulmonary fibrosis is a well-recognized adverse effect in human chemotherapy. Intratracheal (IT) instillation of BL in rodents has been widely used for studying mechanisms of fibrogenesis and for screening potentially desirable antifibrotic compounds. Although the initial cause of BL-induced pulmonary toxicity is attributed to the generation of reactive oxygen species (ROS) once it binds to iron and DNA, the process leading to the final manifestation of pulmonary fibrosis involves release of various inflammatory mediators (Giri and Wang., Comments Toxicol., 3: 145-176 (1989)). The pathogenesis of BL-induced lung injury is initially ushered in by edema, hemorrhage, and a cellular infiltrate predominated by neutrophils and macrophages. An excess accumulation of the inflammatory leukocytes in vascular, interstitial and alveolar spaces of the lung could inflict vasculai- and parenchymal injury by the generation of ROS and proteolytic enzymes. Neutrophils contain substantial amounts of myeloperoxidase (MPO), which can oxidize Cl- to hypochlorous acid (HOCI) in a reaction with H2O2 and the neutrophil-derived HOCI is known to cause cellular toxicity. Macrophage and neutrophil derived ROS is able to stimulate the production of proinflammatory and fibrogenic cytokines that mediate enhanced fibroproliferative response (Phan and Kunkel., Exp. Lung Res., 18: 29-43 (1992)). Besides a large inflammatory cell infiltration, the fibrotic process is further characterized by a hyperproliferative response of activated fibroblasts. The fibroblast-like cells are primarily responsible for an absolute increase in lung collagen content, and an abnormality in the ultrastructural appearance and spatial distribution of collagen types.

### Example 2:

### Inhibition of Fibrosis with antagonist to alpha4 subunit containing Integrin

### Materials and methods

A non-specific control antibody (1E6) and antibody against alpha4 subunit containing integrins (PS2) were used. 1E6 is a mouse anti-human LFA3 (domain 1) IgG1 monoclonal antibody. See Miller, Hochman, Meier, Tizard, Bixler, Rosa, and Wallner (1992) J. Exp. Med. 178: 211-222. PS/2 is produced by the method of Miyake et al., J. Exp. Med., 173: 599-607 (1991).

Chronic respiratory disease-free male C57BL/6 mice weighing 25-30 g were purchased from Charles River Laboratory. Bleomycin sulfate (trademarked as Blenoxane) was a gift from Bristol Laboratories, (Syracuse, NY). The L-[3,4- 3 H]proline for labeling the procollagen substrate of prolyl hydroxylase was obtained from NEN Life Science Products (Boston, MA). Z-fix, an aqueous buffered zinc formalin, was purchased from Anatech, LTD (Battle Creek, MI). All other reagents were of reagent grade or higher purity and obtained from standard commercial sources.

### Treatment of animals.

Mice were housed 4 per cage and cared in accordance with NIH's Guidelines for Animal Welfare. The mice were allowed to acclimate in the facilities for one week prior to all treatments. A 12 h/12 h light/dark cycle was maintained and had access to water and Rodent Laboratory Chow *ad lib*. Animals were randomly divided into 4 experimental groups: 1) SA + SA; 2) BL + IE6; 3) BL + SA; and 4) BL + PS2. Mice were intratracheally (IT) injected with a single dose of saline or BL at 0.08 units/100 microliter/mouse under xylazine and ketamine anesthesia. After IT instillation, the mice received IP injection of IE6, SA or PS2 (100 microgram in 0.2ml/mouse) three times a week. Twenty one days after the BL instillation, mice were sacrificed under pentobarbital anesthesia for bronchoalveolar lavage fluid (BALF), biochemical and histopathological analyses.

### Preparation of BALF and lung tissue.

After anesthesia, the abdominal cavity was opened followed by exsanguination of the descending abdominal aorta. The lungs were prepared for lavage by cannulating the trachea with blunt needle attached to a syringe. The lung lavage was carried out with 3 ml of cold isotonic saline delivered in 1-ml aliquots. An aliquot of the BALF was portioned for total cell count. The remaining BALF was centrifuged at 1500g for 20 min at 4 degrees C, the resulting supernatant aliquoted and then stored at -70 degrees C. After BALF, the lung lobes were quickly dissected free of non-parenchymal tissue, immediately frozen in liquid nitrogen and stored at -70 degrees C.

Later, the frozen lungs were thawed and homogenized in 0.1 M KCI, 0.02 M Tris (pH 7.6) with a Polytron homogenizer (Brinkmann Instruments Inc., Westbury, NY). The homogenate was thoroughly mixed by repeated inversions and the final homogenate volumes (4-5 ml) were recorded. The homogenate was divided into several aliquots and stored at -70 degrees C for biochemical measurements.

### Determination of lung malondialdehyde equivalent and hydroxyproline content.

Lung malondialdehyde equivalent was estimated from the total amount of thiobarbituric acid-reacting products in unfractionated homogenate by the method of Ohkawa *et al*., Anal. Biochem., 95: 351 (1979). For lung hydroxyproline assay, 1 ml of homogenate was precipitated with 0.25 ml ice cold 50% (w/v) trichloroacetic acid, centrifuged and the precipitate hydrolyzed in 2 ml of 6 N HCl for 18 hours at 110 degrees C. The hydroxyproline content was measured by the technique described by Woessner, J.F., Arch. Biochem. Biophys., 93: 440 (1961).

### Determination of prolyl hydroxylase (EC 1.14.11.2) activity.

The preparation of prolyl hydroxylase substrate (procollagen) and the method for prolyl hydroxylase assay are described in Giri, S.N. et al., Exp. Mol. Pathol. 39: 317 (1983). Briefly, tibias freshly isolated from ten-day old chicken embryos were labeled with [3H]-proline in proline-free culture medium at 37 degrees C for 6 h. After removing the unincorporated label by washing, the tissue was homogenated and centrifuged at 3000 g for 20 min at 4 degrees C. The resulting supernatant was dialyzed extensively to remove the unincorporated label. The labeled procollagen substrate was aliquoted and stored at -70 degrees C. The incubation mixture for the enzyme assay in a total volume of 2 ml consisted of ferrous ammonium sulphate (0.1 mmol/l), alphac-ketoglutaric acid (0.1 mmol/l), [3 H] proline procollagen (200,000 dpm), lung homogenate (0.2 ml), ascorbic acid (0.5 mmol/l), and TRIS-hydrochloric acid buffer (0.1 mol/l, pH 7.8). The reaction adding 0.2 ml of 50% trichloroacetic acid after 30 min at 37 degrees C in a Dubnoff metabolic shaker. During the reaction, tritiated water is released in stoichiometric proportion to prolyl hydroxylation and it is used as a measure of the enzyme activity. The tritiated water of the reaction system was separated by vacuum distillation of the whole reaction mixture and counted for radioactivity. The enzyme activity was expressed as dpm of triated water released per total lung per 30 min.

### Determination of cell counts in BALF.

The total cell number in the BALF was determined as described in Wang, Q. et al., Lab. Invest., 67: 234-242 (1992). Total leukocyte number in the BALF was estimated by Coulter counter (Model F, Coulter Electronics, Inc., Hialeah, FL), according to the User's Manual.

### BALF protein assay.

Protein content in the BALF supernatant was determined using the Bio-Rad protein assay (Bio-Rad Laboratories, Richmond, CA) and bovine serum albumin was used as the standard.

### Histopathologic and immunohistochemical analysis.

Three to four animals from each treatment group were randomly chosen for histopathologic and immunohistochemical evaluation at the end of the experiment. The abdominal cavity of the animal was opened followed by exsanguination of the descending abdominal aorta. Immediately thereafter, the lung tissue was prepared for histologic analysis as described in Wang et al, above. After cannulating the trachea with a blunt needle, the thoracic cavity was opened, and then both heart and lung were removed *en bloc.* The lungs were fixed with Z-fix solution via the trachea at a pressure of 30 cm of water. The right cranial and caudal lobes and the left lobe were later blocked, embedded in paraffin, cut in 7-micron sections and stained with hematoxylin and eosin. For immunohistochemical staining for alphaSMA, the lung tissue sections were deparaffinized and endogenous peroxidase was blocked. Sections to be stained were then treated with blocking goat serum for 30 min, and were incubated for 16 h with the primary monoclonal anti-alphaSMA antibody (Sigma Chemical Co., St Louis, MO).

### Statistical analysis of data.

Animal data were expressed on the basis of per total lung and were reported as the mean ± standard error (SE). The data were compared within the four groups using two-way analysis of variance (SIGMASTAT) and Student-Newman-Keuls method. A value of P ≤ 0.05 was considered significant.

### RESULTS

### Lipid peroxidation in the mouse lung.

Lung malondialdehyde equivalent content as an index of lipid peroxidation was assaued various groups of mice. BL instillation significantly increased malondialdehyde equivalent content in mouse lungs in both BL+IE6 and BL+SA groups as compared with SA+SA and BL+PS2 groups (data not presented). The treatment with PS2 effectively blocked BL-induced lung lipid peroxidation since the lung malondialdehyde equivalent level in BL+PS2 group is no different from that of the SA+SA group.

### Lung hydroxyproline content in the mouse lung.

Lung hydroxyproline, the key index of lung collagen level was determined for the 4 groups of mice. The IT instillation of BL significantly elevated the lung hydroxyproline level in the BL+IE6 and BL+SA groups to 185% and 205% of SA+SA control group, respectively. The BL-induced increase in the lung hydroxyproline level in BL+PS2 group was decreased significantly by 35% by the treatment with PS2 compared to the BL+SA group. The lung hydroxyproline level in BL+TR group was not significantly higher than that of the IT SA control (SA+SA) group.

### Prolyl hydroxylase activity in the mouse lung.

The prolyl hydroxylase activities in the lungs of various groups showed that BL alone significantly increased the lung prolyl hydroxylase activity in the BL+SA group to 207% of SA+SA control group. PS2 significantly decreased the prolyl hydroxylase activity elevated by the BL treatment in the BL+SA group.

### Total BALF cell counts in the mouse lung.

The numbers of total cells in the BALF of various groups at 21 days after IT instillation of saline or BL showed that BL treatment increased the total cell counts in the BALF from the BL+IE6 and BL+SA groups compared with the saline control group (SA+SA), although only the BL+IE6 group had significantly higher levels than the SA+SA group. The BALF cell count in BL+PS2 group was not different from that of SA+SA group.

### Protein content in the BALF.

The protein content of the BALF supernatant for the 4 experimental groups revealed that IT instillation of BL significantly increased the BALF protein of all the BL treated groups as compared with SA+SA group. However, treatment with PS2 in BL+PS2 group decreased the BL-induced increase in the BALF supernatant protein, although the difference was not statistically significant.

### Histopathology of the mouse lung.

Histological examination of the mouse lungs revealed normal pulmonary parenchymal tissue in SA+SA group. However, the lungs from the BL+IE6 and BL+SA groups showed a patchy alveolitis and multifocal interstitial fibrosis containing an accumulation of extracellular fibers. The lungs of mice in these groups had thickened interalveolar septa and inflammatory cells in adjacent airspaces. Compared to BL+IE6 and BL+SA groups, lungs from the BL+PS2 group had much less fibrotic lesions, although some lobes still showed a mild degree of interstitial fibrosis.

### Immunohistochemical staining for alphaSMA in the mouse lung.

To determine the accumulation of fibroblasts and fibroblast-like cells in mice after BL treatment, we examined the expression of (XSMA in the lung tissue using a monoclonal antibody against (XSMA. In the control lungs, the immunopositivity occurred in the vascular and bronchial smooth muscle layers. In the BL and control antibody or saline treated lungs, there was extensive and intense immunostaining within fibrotic areas, both in the interstitium and pleura. However, the BL and PS2 treated lungs showed much reduced immunostaining of alphaSMA, compared to BL+IE6 and BL+SA groups.

### Discussion

IPF is a crippling disease and responds poorly to current therapy. In the present study, we provide evidence that alpha4 subunit containing integrin is other possible target in managing IPF. It is generally assumed that the leukocytes of the lung are involved in the evolution of pulmonary fibrosis by the secretion of ROS, fibrogenic cytokines and growth factors. The traffic and state of activation of leukocytes are modulated by various surface proteins such as the integrins. It is clear that cell-cell interactions as well as cell-ECM interactions are critical for the pathogenesis of pulmonary fibrosis. A consistent finding in patients with active pulmonary fibrosis and in animal models of fibrotic lung diseases is the accumulation of increased numbers of immune and inflammatory cells in areas undergoing fibrosis.

VLA-4 is expressed on all circulating leukocytes, and binds to vascular cell adhesion molecule (VCAM-1), a member of the Ig gene superfamily that is expressed on cytokine activated endothelial cells, and to the matrix protein fibronectin. Alpha4beta7 is expressed on a subset of T and B cells, natural killer cells, and eosinophils. It binds to the mucosal vascular addressin (MAdCAM-1), a member of the Ig and mucin-like families of adhesion molecules, as well as to VCAM-1 and fibronectin. Studies in vitro have demonstrated that VLA-4 interaction with VCAM-1 is involved in mononuclear leukocyte and eosinophil adherence to endothelium and transendothelial migration and alpha4beta7 is thought to be involved primarily in leukocyte recruitment to gut associated lymphoid tissue.

In the present study, treatment with PS2 decreased BL-induced increases in total leukocytes in the BALF. The decreased leukocytes in the lungs of BL+PS2 mice may be responsible for the diminished inflammatory injury and fibrosis in the lungs of BL-treated animals. The BL-induced lung injury was significantly reduced by the treatment of PS2 as indicated by measuring the lung lipid peroxidation. The increased collagen in the lung has been associated with increased numbers of fibroblasts in the interstitium and in the alveolar space itself. Many of these fibroblast-like cells are myofibroblasts which have a distinctive phenotype that includes the expression of alphaSMA, a contractile protein commonly found in smooth muscle cells and thought to be important in fibrogenesis and wound healing. A significant finding of the present study was that PS2 treatment attenuated the BL-induced myofibroblast proliferation. Without wishing to be bound by any particular theory, administration with antibody against alpha integrin may have reduced the level of growth factors in the lung released by infiltrating leukocytes or have directly affected the behavior of myofibroblasts. In either case, the reduced proliferating myofibroblasts could lead to a decrease in the lung collagen accumulation in the BL-treated animals in BL+PS2 group.

### EXAMPLE 3:

### Inhibition of Fibrosis with antagonist to alpha1 subunit containing Integrin

### Treatment of Animals

Male C57/BL6 mice weighing 28-30 g, are housed in plastic cages in groups of 4 in facilities approved by the American Association for Accreditation of Laboratory Animal Care. The animals were allowed to acclimate for one week to laboratory conditions prior to starting the experiments. They had access to Rodent Laboratory Chow 5001 (Purina Mills, Inc., St. Louis, MO) and water *ad libitum* and housed in a room which gets filtered air and has 12hr light/12hr dark cycle. Mice were assigned into the following groups :

| GROUP | TREATMENT |
|---|---|
| A | Saline + Phosphate buffered saline |
| B | Saline + control antibody IgG |
| C | Bleomycin + control antobody IgG |
| D | Bleomycin + anti-α1 β1 integrin antibody ) |
| | |

Bleomycin sulfate is dissolved in pyrogen free sterile isotonic saline just before intratraceheal (IT) instillation. Under methoxyflurane anaesthesia mice in appropriate groups received by intratracheal administration either 100 µl of sterile isotonic solution or 0.08 units of bleomycin solution in 100 µl. Antibodies (4 mg/kg) are administered by intraperitoneal injection to mice in appropriate groups three times a week for 21 days post installation. Thereafter, the animals in each group were killed by an overdose of sodium pentobarbital (100-125 mg/kg ip) and their lungs processed for bronchoalveolar lavage, biochemical and histopathological studies.

### Determination of total cell number and protein levels in broncoalveolar lavage

After cannulation of the trachea the lungs are lavaged with 5 ml of isotonic saline, given in five aliquots of 1 ml. The saline is administered with a syringe through the cannula, the chest wall was gently massaged, and the fluid withdrawn. The fluid is centrifuged at 1500 g for 20 minutes at 4 degree C, and resuspended in isotonic saline solultion. The protein content for the supernatant from broncoalveolar lavage specimens is detemined by a method of Lowry et al., J Biol. Chem. 1193: 265-275 (1951), with bovine serume albumin as a standard. Total leukocyte count of cells in suspension is determined in a Coulter Counter (Coulter Electronics, Hialeah, FL).

### Determination of Hydoxyproline

The lungs of animals used for biochemical studies are perfused *in situ* via the right ventricle with ice-cold isotonic saline to wash out blood from the pulmonary vasculature through an opening in the left auricle. The lung lobes are quickly dissected free of non-parenchymal tissue, dropped in liquid nitrogen for quick freezing and then stored at -80C. The frozen lungs are later thawed and homogenized in 0.1 M KCl, 0.02 M Tris buffer (pH 7.6) with a Polytron homogenizer. Hydroxyproline content of the lung homogenate as a measure of collagen content is quantitated by the techniques of Woessner, Arch. Biochem. Biophys. 93: 440-447 (1961).

### Histopathological Study

After lung lavage, the thoracic cavity is opened and the heart and lungs removed *en bloc*. The lungs are instilled with a 1% glutaraldehyde-paraformaldehyde fixative in 0.12M cacodylate buffer at 400m Osm at 30 cm H₂0 presure. The lungs are fixed via this pressure for about 2 hours and then stored in fixative with the tracheas occluded. Before embedding, the lung is isolated from the heart and all non-pulmonary tissue by blunt dissection and removed. Blocks of tissue are cut from at least two sagittal slabs (2-3mm thick) from the right cranial, right caudal, and left lung lobes of each lung. Each block is cut with about a 1 cm² face. The blocks are dehydrated in a graded series of ethanol and embedded in paraffin. Sections (5µm thick) are cut from the paraffin blocks and stained with haematoxylin and eosin for histological evaluations.

### Data Analysis and Interpretation

The data are analyzed in terms of average values with their standard deviations and standard errors of means. Student's t-test, chi-square distributions, correlation coefficient, analysis of variance (ANOVA) and multiple comparison test are applied to judge the significance of differences between the control and treatment groups using a computer based statistical package (SAS/STAT Guide, 6th Ed. Cary, N.C. pp. 183-260 (1985)).

### Results

In this study, we test the hypothesis that neutralizing antibody for integrin α1β1 (antiα1β1) reduces bleomycin (BL)-induced lung fibrosis *in vivo.* Male C57/BL6 mice are intratraceally (IT) injected saline (SA) or BL at 0.08 U in 0.1ml followed by intraperitoneal (IP) injection of the antibody (100 µg in 0.2ml) three times a week. Twenty-one days after the IT instillation, mice are killed for bronchoalveolar lavage (BAL), biochemical and histopathological analysis.

### Histopathological Examination of Lungs

We expect that mice treated with saline and control IgG have no visible lesions and displayed interalveolar septa with a normal thin appearance. In contrast, mice treated with bleomycin and control IgG will have lesions varying from multifocal locations in proximal acini to a diffuse distribution that occasionally involved the pleura. We expect that the lungs of mice treated with bleomycin and anti-alpha1 integrin antibodies appear more like those in Group B (above). We expect Group D animals to exhibit only a limited number of fibrotic lesions, with mild multifocal septal thickening and small aggregates of mononuclear cells.

Although the foregoing invention has been described in some detail by way of illustration and example for purposes of clarity of understanding, it will be apparent to those skilled in the art that certain changes and modifications will be practiced. Therefore, the description and examples should not be construed as limiting the scope of the invention, which is delineated by the appended claims.

## Claims

1. Use of a composition comprising an antibody homolog which is an antagonist of an interaction between an alpha4 subunit-bearing integrin and a ligand for an alpha4 subunit-bearing integrin for the preparation of a pharmaceutical composition for the treatment of fibrosis in a subject.

2. The use of claim 1, wherein the fibrosis is fibrosis of an internal organ.

3. The use of claim 2, wherein the internal organ is the liver, lung, kidney, heart blood vessels, or gastrointestinal tract.

4. The use of claim 1, wherein the subject suffers from pulmonary fibrosis, myelofibrasis, liver cirrhosis, mesangial proliferative glomerulonephritis, crescentic glomerulonephritis, diabetic nephropathy, renal interstitial fibrosis, or HIV associated nephropathy.

5. The use of claim 1, wherein the fibrosis is dermal fibrosis.

6. The use of claim 5, wherein the subject suffers from scleroderma, morphea, keloids, hypertrophic scars, familial cutaneous collagenoma, or connective tissue nevi of the collagen type.

7. The use of claim 1, wherein the fibrosis is fibrosis of the eye.

8. The use of claim 7, wherein the subject suffers from diabetic retinopathy, postsurgical scarring, or proliferative vitreoretinopathy.

9. The use of any one of the preceding claims 1 to 8, wherein the composition comprises an anti-alpha4 antibody homolog.

10. The use of any one of the preceding claims, wherein the antibody homolog is a humanized, human or chimeric antibody homolog.

11. The use of any one of the preceding claims 1 to 10 wherein the pharmaceutical composition is configured to be administered:
(a) at a dosage of from 0.1 mg/kg per day to 10 mg/kg per day;
(b) over a dosing period of from one to fourteen days; and
(c) in an amount sufficient to maintain a plasma level of the antibody homolog of 1 mg/ml or greater.

12. The use of any one of the preceding claims 1 to 11, wherein the antibody homolog is a humanized mouse 21-6 antibody.

13. The use of any one of the preceding claims 1 to 12, wherein the subject is a human.

## Patentansprüche

1. Verwendung einer Zusammensetzung, die ein Antikörper-Homolog umfasst, das ein Antagonist einer Interaktion zwischen eine alpha4-Untereinheit tragendem Integrin und einem Liganden für eine alpha4-Untereinheit tragendes Integrin ist, für die Herstellung eines Arzneimittels zur Behandlung von Fibrose in einem Individuum.

2. Verwendung nach Anspruch 1, wobei die Fibrose die Fibrose eines inneren Organs ist.

3. Verwendung nach Anspruch 2, wobei das innere Organ die Leber, Lunge, Niere, Blutgefäße des Herzens oder der Gastrointestinaltrakt ist.

4. Verwendung nach Anspruch 1, wobei das Individuum an Lungenfibrose, Myelofibrose, Leberzirrhose, mesangialer proliferativer Glomerulonephritis, rasch progredienter Glomerulonephritis, diabetischer Nephropathie, renaler interstitieller Fibrose oder HIVassoziierter Nephropathie leidet.

5. Verwendung nach Anspruch 1, wobei die Fibrose dermale Fibrose ist.

6. Verwendung nach Anspruch 5, wobei das Individuum an Sklerodermia, Morphea, Wulstnarben, hypertrophischen Narben, familiärem kutanem Kollagenom oder Bindegewebsnaevi vom Kollagentyp leidet.

7. Verwendung nach Anspruch 1, wobei die Fibrose eine Fibrose des Auges ist.

8. Verwendung nach Anspruch 7, wobei das Individuum an diabetischer Retinopathie, postoperativer Narbenbildung oder proliferativer Vitreoretinopathie leidet.

9. Verwendung nach einem der vorhergehenden Ansprüche 1 bis 8, wobei die Zusammensetzung ein anti-alpha4 Antikörper-Homolog umfasst.

10. Verwendung nach einem der vorgehenden Ansprüche, wobei das Antikörper-Homolog ein humanisiertes, menschliches oder chimäres Antikörper-Homolog ist.

11. Verwendung nach einem der vorhergehenden Ansprüche 1 bis 10, wobei das Arzneimittel zusammengesetzt ist, um verabreicht zu werden:
(a) in einer Dosierung von 0,1 mg/kg pro Tag bis 10 mg/kg pro Tag;
(b) über einen Dosierungszeitraum von 1 bis 14 Tagen; und
(c) in einem Umfang, der ausreichend ist, um einen Plasmaspiegel des Antikörper-Homologs von 1 mg/ml oder höher beizubehalten.

12. Verwendung nach einem der vorhergehenden Ansprüche 1 bis 11, wobei das Antikörper-Homolog ein humanisierter Maus 21-6-Antikörper ist.

13. Verwendung nach einem der vorhergehenden Ansprüche 1 bis 12, wobei das Individuum ein Mensch ist.

## Revendications

1. Utilisation d'une composition comprenant un homologue d'anticorps qui est un antagoniste d'une interaction entre une intégrine portant une sous-unité alpha 4 et un ligand d'une intégrine portant une sous-unité alpha 4, pour la préparation d'une composition pharmaceutique pour le traitement de la fibrose chez un sujet.

2. Utilisation selon la revendication 1, dans laquelle la fibrose est une fibrose d'un organe interne.

3. Utilisation selon la revendication 2, dans laquelle l'organe interne est le foie, le poumon, le rein, les vaisseaux sanguins cardiaques ou le tractus gastro-intestinal.

4. Utilisation selon la revendication 1, dans laquelle le sujet est atteint de fibrose pulmonaire, myélofibrose, cirrhose hépatique, glomérulonéphrite proliférante mésangiale (maladie de Berger), glomérulonéphrite en croissant, néphropathie diabétique, fibrose interstitielle rénale, ou néphropathie associée au VIH.

5. Utilisation selon la revendication 1, dans laquelle la fibrose est une fibrose dermique.

6. Utilisation selon la revendication 5, dans laquelle le sujet est atteint de sclérodermie, morphée, chéloïdes, cicatrices hypertrophiées, collagénome cutanée familial, ou nevi de type collagène du tissu conjonctif.

7. Utilisation selon la revendication 1, dans laquelle la fibrose est une fibrose de l'oeil.

8. Utilisation selon la revendication 7, dans laquelle le sujet est atteint de rétinopathie diabétique, cicatrices post-chirurgicales ou vitréo-rétinopathie proliférative.

9. Utilisation selon l'une quelconque des revendications 1 à 8, dans laquelle la composition comprend un homologue d'anticorps anti-alpha 4.

10. Utilisation selon l'une quelconque des revendications précédentes, dans laquelle l'homologue d'anticorps est un homologue d'anticorps humanisé, humain ou chimère.

11. Utilisation selon l'une quelconque des revendications 1 à 10, dans laquelle la composition pharmaceutique est configurée pour être administrée :
(a) à un dosage de 0,1 mg/kg par jour à 10 mg/kg par jour ;
(b) sur une période de dose de un à quatorze jours ; et
(c) en une quantité suffisante pour maintenir un niveau plasmatique de l'homologue d'anticorps de 1 mg/ml ou plus.

12. Utilisation selon l'une quelconque des revendications 1 à 11, dans laquelle l'homologue d'anticorps est un anticorps de souris 21-6 humanisé.

13. Utilisation selon l'une quelconque des revendications 1 à 12, dans laquelle le sujet est un humain.
